(19) Europäisches Patentamt
European Patent Office
Office européen des brevets

(11) EP 0 612 244 B1

# (12) EUROPEAN PATENT SPECIFICATION

(45) Date of publication and mention of the grant of the patent:
**19.09.2001 Bulletin 2001/38**

(21) Application number: **92925061.1**

(22) Date of filing: **29.10.1992**

(51) Int Cl.[7]: **A61K 31/19**, A61K 31/36, A61K 31/41, A61K 31/66, C07C 61/20, C07C 62/32, C07D 257/04, C07D 317/50, C07D 405/08, C07F 9/30, C07D 317/60, C07C 62/34

(86) International application number:
**PCT/US92/09427**

(87) International publication number:
**WO 93/08799 (13.05.1993 Gazette 1993/12)**

(54) **ENDOTHELIN RECEPTOR ANTAGONISTS**

ENDOTHELIN-REZEPTOR-ANTAGONISTEN

ANTAGONISTES RECEPTEURS DE L'ENDOTHELINE

(84) Designated Contracting States:
**AT BE CH DE DK ES FR GB GR IE IT LI LU MC NL SE**

(30) Priority: **05.11.1991 US 787870**
**20.03.1992 US 854195**

(43) Date of publication of application:
**31.08.1994 Bulletin 1994/35**

(73) Proprietor: **SMITHKLINE BEECHAM CORPORATION**
**Philadelphia Pennsylvania 19101 (US)**

(72) Inventors:
- **COUSINS, Russell, Donovan**
**Oxford, PA 19363 (US)**
- **ELLIOTT, John, Duncan**
**Wayne, PA 19087 (US)**
- **LAGO, Maria, Amparo**
**Audubon, PA 19403 (US)**
- **LEBER, Jack, Dale**
**Doylestown, PA 18901 (US)**
- **PEISHOFF, Catherine, Elisabeth**
**West Chester, PA 19380 (US)**

(74) Representative: **Waters, David Martin, Dr.**
**Smithkline Beecham plc**
**Corporate Intellectual Property,**
**Two New Horizons Court**
**Brentford, Middlesex TW8 9EP (GB)**

(56) References cited:
**EP-A- 0 206 241** **US-A- 3 642 785**
**US-A- 3 737 455**

- **TETRAHEDRON LETTERS., no.54, 1969, OXFORD GB pages 4795 - 4796 JACQUELINE FICINI ET AL 'Sur l'obtention d'un réactif de Grignard porteur en béta d'une fonction cétone'**
- **JOURNAL OF ORGANIC CHEMISTRY., vol.25, no.12, December 1960, EASTON US pages 2088 - 2091 C.F. KOELSCH 'Electrophilic properties of ethyl 3-phenylindone-2-carboxylate'**
- **Bulletin of the Chemical Society of Japan, Vol. 59, issued 1959, Tokyo, Japan, K. YAMAMURA et al., "Formation of 2-Substituted 1,3-Diphenylindenes by an N-Bromosuccinimide Prompted Dehydrocyclization of 2-Substituted 1,3,3-Triphenyl-1-propenes", pages 3699-3701.**
- **CHEMICAL ABSTRACTS, Vol. 88, No. 25, published 19 June 1978 (Columbus, Ohio, USA), The Abstract Number 190,677p, M.I. KOMENDANTOV et al., "1,3-Dipolar Cycloaddition of Diphenyldiazomethane to methyl and ethyl esters of phenylpropiolic acid and its nitrile", Tezisy, Dokl. - Vses. Konf. Khim. Atsetilena, 5th, 1975, 374-5.**
- **: "Beilstein Handbook of organic chemistry,G-I,28(6)", 1991, SPRINGER-VERLAG, BERLIN**
- **A. ARCOLEO: "", CHEMISTRY AND INDUSTRY, LONDON, 1976, vol. 15, no. , pages 651 to 651**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to novel indane and indene derivatives, pharmaceutical compositions containing these compounds and their use for the manufacture of a medicament for antagonizing endothelin receptors.

### BACKGROUND

Endothelin (ET) is a highly potent vasoconstrictor peptide synthesized and released by the vascular endothelium. Endothelin exists as three isoforms, ET-1, ET-2 and ET-3. Of these, only ET-1 and ET-3 have been found to be expressed in mammalian systems. [Unless otherwise stated "endothelin" shall mean any or all of the isoforms of endothelin]. Endothelin has profound effects on the cardiovascular system, and in particular, the coronary, renal and cerebral circulation. Elevated or abnormal release of endothelin is associated with smooth muscle contraction which is involved in the pathogenesis of cardiovascular, cerebrovascular, respiratory and renal pathophysiology. Elevated levels of endothelin have been reported in plasma from patients with essential hypertension, acute myocardial infarction, subarachnoid hemorrhage, atherosclerosis, and patients with uraemia undergoing dialysis.

In vivo, endothelin has pronounced effects on blood pressure and cardiac output. An intravenous bolus injection of ET (0.1 to 3 nmol/kg) in rats causes a transient, dose-related depressor response (lasting 0.5 to 2 minutes) followed by a sustained, dose-dependent rise in arterial blood pressure which can remain elevated for 2 to 3 hours following dosing. Doses above 3 nmol/kg in a rat often prove fatal.

Endothelin appears to produce a preferential effect in the renal vascular bed. It produces a marked, long-lasting decrease in renal blood flow, accompanied by a significant decrease in GFR, urine volume, urinary sodium and potassium excretion. Endothelin produces a sustained antinatriuretic effect, despite significant elevations in atrial natriuretic peptide. Endothelin also stimulates plasma renin activity. These findings suggest that ET is involved in the regulation of renal function and is involved in a variety of renal disorders including acute renal failure, cyclosporine nephrotoxicity and chronic renal failure.

Studies have shown that in vivo, the cerebral vasculature is highly sensitive to both the vasodilator and vasoconstrictor effects of endothelin. Therefore, ET may be an important mediator of cerebral vasospasm, a frequent and often fatal consequence of subarachnoid hemorrhage.

ET also exhibits direct central nervous system effects such as severe apnea and ischemic lesions which suggests that ET may contribute to the development of cerebral infarcts and neuronal death.

ET has also been implicated in myocardial ischemia (Nichols et al. Br. J. Pharm. 99: 597-601, 1989 and Clozel and Clozel, Circ. Res., 65: 1193-1200, 1989) coronary vasospasm (Fukuda et al., Eur. J. Pharm. 165: 301-304, 1989 and Lüscher, Circ. 83: 701, 1991) heart failure, proliferation of vascular smooth muscle cells, (Takagi, Biochem & Biophys. Res. Commun.; 168: 537-543, 1990, Bobek et al., Am. J. Physiol. 258:408-C415, 1990) and atherosclerosis, (Nakaki et al., Biochem. & Biophys. Res. Commun. 158: 880-881, 1989, and Lerman et al., New Eng. J. of Med. 325: 997-1001, 1991). Increased levels of endothelin have been shown after coronary balloon angioplasty (Kadel et al., No. 2491 Circ. 82: 627, 1990).

Further, endothelin has been found to be a potent constrictor of isolated mammalian airway tissue including human bronchus (Uchida et al., Eur J. of Pharm. 154: 227-228 1988, LaGente, Clin. Exp. Allergy 20: 343-348, 1990; and Springall et al., Lancet, 337: 697-701, 1991).

Endothelin has been associated with the induction of haemorrhagic and necrotic damage in the gastric mucosa (Whittle et al., Br. J. Pharm. 95: 1011-1013, 1988); Raynaud's phenomenon, Cinniniello et al., Lancet 337: 114-115, 1991); Migraine (Edmeads, Headache, Feb. 1991 p 127); Sepsis (Weitzberg et al., Circ. Shock 33: 222-227, 1991; Pittet et al., Ann. Surg. 213: 262-264, 1991), Cyclosporin-induced renal failure or hypertension (Eur. J. Pharmacol., 180: 191-192, 1990, Kidney Int, 37: 1487-1491, 1990) and endotoxin shock and other endotoxin induced diseases (Biochem, Biophys. Res. Commun., 161: 1220-1227, 1989, Acta Physiol. Scand. 137: 317-318, 1989).

Thus, endothelin receptor antagonists would offer a unique approach toward the pharmacotherapy of hypertension, renal failure, cerebrovascular disease, myocardial ischemia, angina, heart failure, asthma, atherosclerosis, Raynaud's phenomenon, ulcers, sepsis, migraine, glaucoma, endotoxin shock, endotoxin induced multiple organ failure or disseminated intravascular coagulation, cyclosporin-induced renal failure and as an adjunct in angioplasty and prevention of restenosis.

### SUMMARY OF THE INVENTION

This invention comprises indane and indene derivatives represented by Formula (I) and pharmaceutical compositions containing these compounds, and their use as endothelin receptor antagonists which are useful in the treatment of a variety of cardiovascular and renal diseases including but not limited to: hypertension, acute and chronic renal failure, cyclosporine induced nephrotoxicity, stroke, cerebrovascular vasospasm, myocardial ischemia, angina,

heart failure and atherosclerosis.

This invention further provides for the use of the compounds of Formula (I) for the manufacture of a medicament for antagonizing endothelin receptors. Beilstein Handbook of Organic Chemistry, General Compound Index, Vol. 28(6), G-I, Springer Verlag, Berlin, 1991 discloses 1,3-diphenylindene, 1,3-diphenylindane and (cis, cis)-(1RS, 3SR)-1,3-diphenylindane-2-carboxylic acid. C.F. Koelsch et al, J. Org. Chem., 1960, 25(12), 2088-2091 discloses (1RS)-1,3-diphenylindene-2-carboxylic acid. A. Arcoleo *et al,* Chem. Ind. (London), 1967, 15, 651 discloses 1,3-bis (3,4-dimethoxyphenyl)-5,6-dimethoxyindane.

None of these documents disclose indane or indene derivatives as pharmaceuticals or endothelin receptor antagonists.

## DETAILED DESCRIPTION OF THE INVENTION

The compounds of this invention are represented by structural Formula (I):

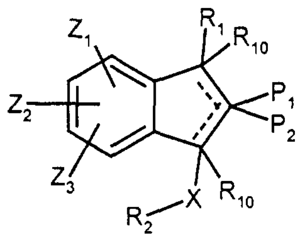

(I)

wherein:

$R_1$ is $X(CH_2)_nAr$, dihydrobenzofuranyl, benzodioxanyl, cyclohexyl, or $C_{1-4}$alkyl;
$R_2$ is a moiety of formula (a) or (b), $C_{1-4}$alkyl, indolyl or hydrogen;
$R_3$ and $R_5$ are independently hydrogen, OH, $C_{1-5}$alkoxy, halogen, $-OC_{1-4}$alkyl phenyl, $R_{11}CO_2R_7$, $C_{1-4}$alkyl, $N(R_6)_2$, $NH(CO)CH_3$, $-X(CH_2)_nR_8$, $-X-R_9-Y$, pyridyl, phenyl or $S(O)_qC_{1-5}$alkyl;
$R_4$ is hydrogen, OH, $C_{1-5}$alkoxy, halogen, $C_{1-4}$alkyl, $N(R_6)_2$, $NH(CO)CH_3$ or $S(O)_qC_{1-5}$alkyl;
$P_1$ and $P_2$ are independently hydrogen, $CO_2H$ or tetrazolyl;
$R_6$ is independently hydrogen or $C_{1-4}$alkyl;
$R_7$ is independently hydrogen, $C_{1-6}$alkyl or $(CH_2)_nAr$;
$R_8$ is $C_{1-8}$alkyl, $C_{2-8}$alkenyl or $C_{2-8}$alkynyl, all of which may be unsubstituted or substituted by one or more OH, $CH_2OH$, $N(R_6)_2$ or halogen; hydrogen, $CO_2H$, $PO_3H_2$, $P(O)(OH)R_7$ or tetrazole;
$R_9$ is $C_{1-10}$alkylene, $C_{2-10}$alkenylene or phenylene, all of which may be unsubstituted or substituted by one or more OH, $N(R_6)_2$, COOH, halogen or $XC_{1-5}$alkyl;
$R_{10}$ is hydrogen;
$R_{11}$ is $C_{1-8}$alkylene, $C_{2-8}$alkenylene or $C_{2-8}$alkynylene, all of which may be unsubstituted or substituted by one or more OH, $CH_2OH$, $N(R_6)_2$ or halogen;
X is $(CH_2)_n$ or oxygen;
Y is $CH_3$ or $-X(CH_2)_nAr$;
Ar is:

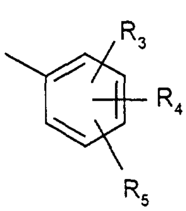

(a),

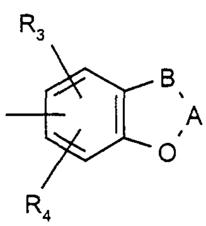

(b),

or pyridyl optionally substituted by one or more $R^3$ or $R^4$ groups;
A is C=O or $[C(R_6)_2]_m$;
B is $-CH_2-$ or -O-;
$Z_1$, $Z_2$ and $Z_3$ are independently $-X-R_9-Y$, benzyl, hydrogen, OH, $C_{1-5}$alkoxy, $-N(R_6)_2$, $S(O)_qC_{1-8}$alkyl, $NHCOR_6$, $X(CH_2)_nR_8$ or halogen, or $Z_1$ and $Z_2$ together may be -O-A-O- on contiguous carbons;
q is zero, one or two;
n is an integer from 0 to six;
m is 1, 2 or 3;and the dotted line indicates the optional presence of a double bond; or a pharmaceutically acceptable salt thereof;

provided that a) when the optional double bond is present there is only one $R_{10}$ and there is no $P_1$; and b) the compound of Formula (I) is not:

(1RS)-1,3-diphenylindene-2-carboxylic acid;
(cis,cis)-(1RS,3SR)-1,3-diphenylindane-2-carboxylic acid;
1,3-diphenylindene;
1,3-diphenylindane; or
1,3-bis(3,4-dimethoxyphenyl)-5,6-dimethoxyindane.

Also included in the invention are pharmaceutically acceptable salt complexes.
All alkyl, alkenyl, alkynyl and alkoxy groups may be straight or branched. The term "halogen" is used to mean iodo, fluoro, chloro or bromo. Alkyl groups may be substituted by one or more halogens up to perhalogenation.
The compounds of the present invention may contain one or more asymmetric carbon atoms and may exist in racemic and optically active form. All of these compounds and diastereoisomers are contemplated to be within the scope of the present invention.
Preferred compounds are those wherein $R_3$ is hydrogen, $-X(CH_2)_nR_8$ or $R_{11}CO_2R_7$; $R_4$ and $R_5$ are independently hydrogen, OH, $C_{1-5}$alkoxy, $SC_{1-5}$alkyl, F, Br, $C_{1-3}$alkyl or $NH_2$; and $Z_1$ and $Z_3$ are hydrogen and $Z_2$ is hydrogen, OH, $C_{1-5}$alkoxy, halogen, $X(CH_2)_nR_8$, $NH_2$, benzyl, $NH(CO)CH_3$, or $Z_1$ and $Z_2$ together may be O-A-O on contiguous carbons.
Most preferred are compounds wherein $R_1$ is (b) and $R_2$ is (a) or (b); A is $CH_2$, B is -O-; there is no optional double bond; $R_1$ and $XR_2$ are trans to $P_1$; $Z_2$ is OH, $C_{1-5}$alkoxy, $-OCH_2CH=CH_2$ or hydrogen, $Z_1$ is hydrogen; $R_3$ is hydrogen, $X(CH_2)_nCOOH$ or $CH=CHCO_2H$, $R_4$ is hydrogen or $C_{1-2}$alkoxy; and $R_5$ and $P_2$ are hydrogen.
Especially preferred are the following compounds:

(1RS, 2SR, 3SR)-1-(4-Methoxyphenyl)-3-(3,4-methylenedioxyphenyl)indane-2-carboxylic acid;

(1RS, 2RS, 3SR)-5-Hydroxy-3-(4-methoxyphenyl)-1-(3,4-methylenedioxyphenyl)indane-2-carboxylic acid;

(1RS, 2RS, 3SR)-5-Methoxy-3-(4-methoxyphenyl)-1-(3,4-methylenedioxyphenyl)indane-2-carboxylic acid;

(1RS, 2SR, 3SR)-1,3-Bis(3,4-methylenedioxyphenyl)-5-5-hydroxyindane-2-carboxylic acid;

(1RS,2SR,3RS)-3-(2-Carboxymethoxy-4-methoxyphenyl)-1-(3,4-methylenedioxyphenyl)-5-(prop-1-yloxy)-indane-2-carboxylic acid

(1RS, 2SR, 3SR)-3-(2-Carboxymethoxy-4-methoxyphenyl)-1-(2-methoxy-4,5-methylenedioxyphenyl)-5-(prop-1-yloxy)-indane-2-carboxylic acid

(1RS, 2SR, 3RS)-3-[2-(1-Carboxyeth-2-yloxy)-4-methoxy-phenyl]-1-(3,4-methylenedioxyphenyl)-5-(prop-1-yloxy) indane-2-carboxylic acid, bis-dicyclohexylamine salt;

(1RS, 2SR, 3SR)-3-[2-[(E)-2-Carboxyethen-l-yl]-4-methoxyphenyl]-1-(3,4-methylenedioxyphenyl)-5-(prop-1-yloxy)indane-2-carboxylic acid;

(1RS, 2SR, 3SR)-3-[2-(2-Carboxyeth-1-yl)-4-methoxyphenyl]-1-(3,4-methylenedioxyphenyl)-5-(prop-1-yloxy)indane-2-carboxylic acid;

(1RS,2SR,3RS)-3- [2-(3-Carboxyphenyl)-4-methoxyphenyl]-1-(3,4-methylenedioxyphenyl)-5-(prop-1-yloxy)indane-2-carboxylic acid.

The present invention provides compounds of Formula (I) above

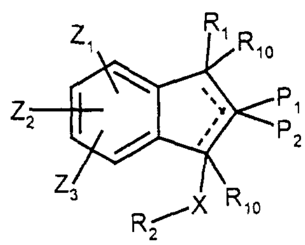

(I)

which can be prepared by a process which comprises:

a) reacting a compound of formula (2) wherein X is $C_{1-5}$alkyl

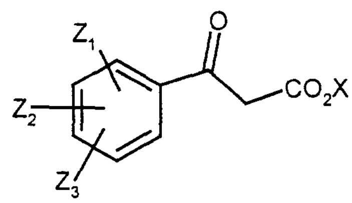

(2)

with a substituted benzaldehyde or aldehyde of formula (3):

D-CHO (3)

wherein D is Ar or (c) as defined in Formula (I), in a suitable solvent such as benzene with a catalyst such as piperidinium acetate at reflux to provide a compound of formula (4):

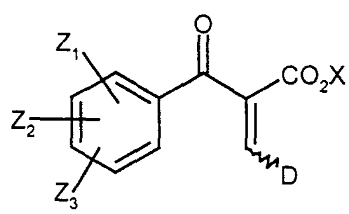

(4)

Cyclization of compound (4) in the presence of a suitable Lewis acid such as titanium tetra-choloride or aluminum chloride or alternatively when $Z_1$ is 3-OR (meta)(where R is $C_{1-5}$alkyl, or benzyl), trifluoroacetic acid, provides an indanone of the formula (5):

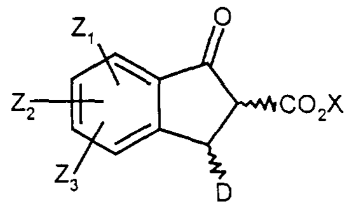

(5)

Dehydrogenation with 2,3-dichloro-5,6-dicyano-1,4-benzoquinone in an appropriate solvent or alternatively bromination with pyridinium hydrobromide perbromide in dichloromethane followed by treatment with 1,5-diazabicyclo[4,3,0]non-5-ene provides indenones of formula (6):

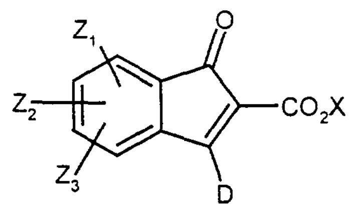

(6)

b) Alternatively, a compound of formula (6) wherein $Z_1$, $Z_2$ and $Z_3$ are hydrogen and

D = 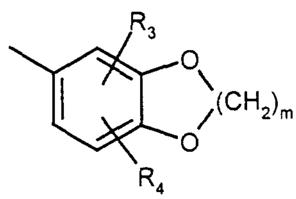

can be prepared by treatment of 2-bromobenzoic acid with two equivalents of n-butyllithium in a solvent such as tetrahydrofuran under argon at -78°C followed by the addition of an acid chloride of formula (7):

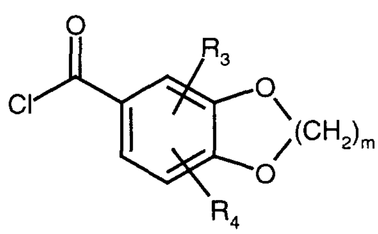

(7)

provides a compound of formula (8):

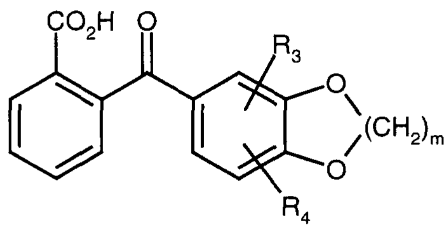

(8)

Treatment of compounds of type (8) with thionyl chloride at reflux gives an acid chloride which can be isolated by concentration under reduced pressure. This acid chloride can then be treated with diethyl magnesium malonate in a solvent such as ether to give a compound of formula (9):

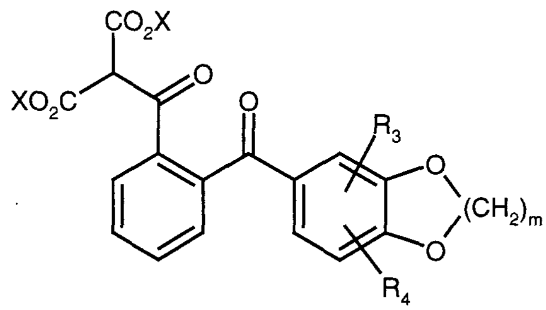

(9)

Reaction of a compound of type (9) at reflux with 5% aqueous sodium carbonate gives compounds of formula (10):

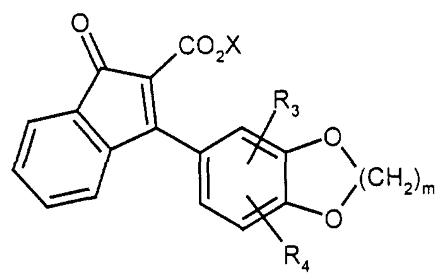

(10)

c) Treatment of an indenone of formula (11):

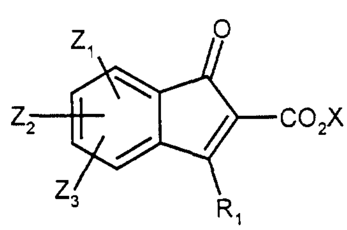

(11)

wherein $Z_1$, $Z_2$, $Z_3$ and $R_1$ are as defined for Formula I or a group convertable to them, with an organomagnesium compound of formula (12):

$$R_2(CH_2)_nMgBr \quad (12)$$

wherein $R_2$ is defined for Formula (I) or a group convertable to it, in a suitable solvent at 0°C provides compounds of formula (13):

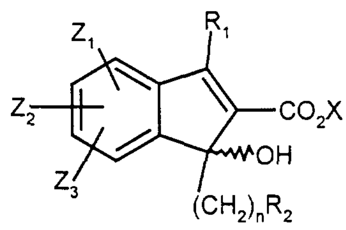

(13)

Saponification of compounds of formula (13) using sodium hydroxide in aqueous methanol followed by reduction with triethylsilane and boron trifluoride etherate in a suitable solvent such as dichloromethane at 0°C affords racemic compounds of formula (14):

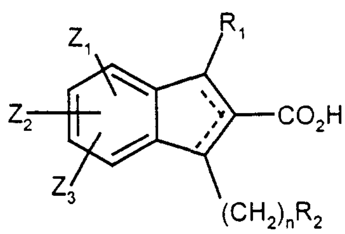

(14)

Conjugate addition of nucleophiles to an ester derived from formula (14), followed by saponification affords compounds of formula (I) having an $R_{10}$ other than hydrogen. Re-introduction of a double bond into an ester derived from such acids followed by conjugate addition of another nucleophilic species and subsequent saponification affords compounds of formula (1) in which neither $R_{10}$ substituent is hydrogen.

Reduction of compounds of formula (13) with triethylsilane and boron trifluoride etherate in a suitable solvent such as dichloromethane at 0°C followed by hydrogenation with hydrogen gas under pressure at approximately 60 psi in the presence of a suitable catalyst such as 10% palladium on charcoal affords compounds of formula (15):

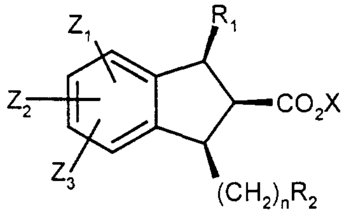

(15)

Alkylation or acylation of the ester enolate derived from formula (15) affords compounds wherein $P_1$ and $P_2$ are as defined in Formula (I).

Alternatively, hydrogenation of compounds of formula (13) with hydrogen gas under pressure at approximately 60 psi in the presence of a suitable catalyst such as 10% palladium on charcoal in a suitable solvent such as ethyl acetate or methanol containing 1-5% acetic acid affords compounds of formula (15). Treatment of these compounds with a base such as sodium hydroxide in a suitable solvent such as aqueous ethanol provides racemic compounds of formula (16):

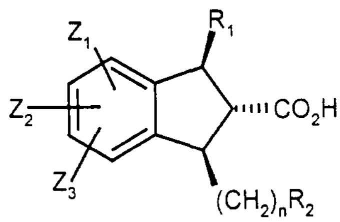

(16)

wherein $Z_1$, $Z_2$ and $Z_3$ are hydrogen; $R_1 = R_2$; and n is 0.
Treatment of compounds of formula (13) with triethylsilane and boron trifluoride etherate in a suitable solvent such as dichloromethane at 0°C followed by reaction with samarium II iodide in a suitable solvent such as tetrahydrofuran and then saponification, provides compounds of formula (17)

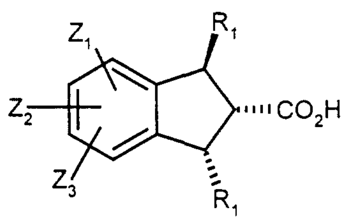

(17)

With appropriate manipulation and protection of any chemical functionalities, synthesis of the remaining compounds of the Formula (I) is accomplished by methods analogous to those above and to those described in the Experimental section.

In order to use a compound of the Formula (I) or a pharmaceutically acceptable salt thereof for the treatment of humans and other mammals it is normally formulated in accordance with standard pharmaceutical practice as a pharmaceutical composition.

Compounds of Formula (I) and their pharmaceutically acceptable salts may be administered in a standard manner for the treatment of the indicated diseases, for example orally, parenterally, sublingually, transdermally, rectally, via inhalation or via buccal administration.

Compounds of Formula (I) and their pharmaceutically acceptable salts which are active when given orally can be formulated as syrups, tablets, capsules and lozenges. A syrup formulation will generally consist of a suspension or solution of the compound or salt in a liquid carrier for example, ethanol, peanut oil, olive oil, glycerine or water with a flavouring or colouring agent. Where the composition is in the form of a tablet, any pharmaceutical carrier routinely used for preparing solid formulations may be used. Examples of such carriers include magnesium stearate, terra alba, talc, gelatin, agar, pectin, acacia, stearic acid, starch, lactose and sucrose. Where the composition is in the form of a capsule, any routine encapsulation is suitable, for example using the aforementioned carriers in a hard gelatin capsule shell. Where the composition is in the form of a soft gelatin shell capsule any pharmaceutical carrier routinely used for preparing dispersions or suspensions may be considered, for example aqueous gums, celluloses, silicates or oils and are incorporated in a soft gelatin capsule shell.

Typical parenteral compositions consist of a solution or suspension of the compound or salt in a sterile aqueous or non-aqueous carrier optionally containing a parenterally acceptable oil, for example polyethylene glycol, polyvinylpyrrolidone, lecithin, arachis oil, or sesame oil.

Typical compositions for inhalation are in the form of a solution, suspension or emulsion that may be administered as a dry powder or in the form of an aerosol using a conventional propellant such as dichlorodifluoromethane or trichlorofluoromethane.

A typical suppository formulation comprises a compound of Formula (1) or a pharmaceutically acceptable salt thereof which is active when administered in this way, with a binding and/or lubricating agent, for example polymeric glycols, gelatins, cocoa-butter or other low melting vegetable waxes or fats or their synthetic analogues.

Typical transdermal formulations comprise a conventional aqueous or non-aqueous vehicle, for example a cream, ointment, lotion or paste or are in the form of a medicated plaster, patch or membrane.

Preferably the composition is in unit dosage form, for example a tablet, capsule or metered aerosol dose, so that the patient may administer to themselves a single dose.

Each dosage unit for oral administration contains suitably from 0.1 mg to 500 mg/Kg, and preferably from 1 mg to 100 mg/Kg, and each dosage unit for parenteral administration contains suitably from 0.1 mg to 100 mg, of a compound of Formula (I) or a pharmaceutically acceptable salt thereof calculated as the free acid. Each dosage unit for intranasal administration contains suitably 1-400 mg and preferably 10 to 200 mg per person. A topical formulation contains suitably 0.01 to 1.0% of a compound of Formula (I).

The daily dosage regimen for oral administration is suitably about 0.01 mg/Kg to 40 mg/Kg, of a compound of Formula (I) or a pharmaceutically acceptable salt thereof calculated as the free acid.
The daily dosage regimen for parenteral administration is suitably about 0.001 mg/Kg to 40 mg/Kg, of a compound of the Formula (I) or a pharmaceutically acceptable salt thereof calculated as the free acid. The daily dosage regimen for intranasal administration and oral inhalation is suitably about 10 to about 500 mg/person.
The active ingredient may be administered from 1 to 6 times a day, sufficient to exhibit the desired activity.

No unacceptable toxicological effects are expected when compounds of the invention are administered in accordance with the present invention.

The biological activity of the compounds of Formula (I) are demonstrated by the following tests:

I. Binding Assay

A) Membrane Preparation

Rat cerebellum or kidney cortex were rapidly dissected and frozen immediately in liquid nitrogen or used fresh. The tissues, 1-2 g for cerebellum or 3-5 g for kidney cortex, were homogenized in 15 mls of buffer containing 20mM Tris HCl and 5mM EDTA, pH 7.5 at 4°C using a motor-driven homogenizer. The homogenates were filtered through cheesecloth and centrifuged at 20,000 x g for 10 minutes at 4°C. The supernatant was removed and centrifuged at 40,000 xg for 30 minutes at 4°C. The resulting pellet was resuspended in a small volume of buffer containing 50 mM Tris, 10 mM $MgCl_2$, pH 7.5; aliquotted with small vials and frozen in liquid nitrogen. The membranes were diluted to give 1 and 5 mg of protein for each tube for cerebellum and kidney cortex in the binding assay.

Freshly isolated rat mesenteric artery and collateral vascular bed were washed in ice cold saline (on ice) and lymph nodes were removed from along the major vessel. Then, the tissue was homogenized using a polytron in buffer containing 20 mM Tris and 5mM EDTA, pH 7.5 at 4°C in 15 ml volume for ~6 gm of mesenteric artery bed. The homogenate was strained through cheesecloth and centrifuged at 2,000 xg for 10 min. at 4°C. The supernatant was removed and centrifuged at 40,000 xg for 30 min. at 4°C. The resulting pellet was resuspended as explained above for cerebellum and kidney cortex. Approximately 10 mg of membrane protein was used for each tube in binding experiments.

B) [$^{125}$I]ET-1 Binding Protocol

[$^{125}$I]ET-1 binding to membranes from rat cerebellum (2-5 mg protein/assay tube) or kidney cortex (3-8 mg protein/assay tube) were measured after 60 minutes incubation at 30°C in 50 mM Tris HCl, 10 mM $MgCl_2$, 0.05% BSA, pH 7.5 buffer in a total volume of 100 ml. Membrane protein was added to tubes containing either buffer or indicated concentration of compounds. [$^{125}$I]ET-1 (2200 Ci/mmol) was diluted in the same buffer containing BSA to give a final concentration of 0.2-0.5 nM ET-1. Total and nonspecific binding were measured in the absence and presence of 100 nM unlabelled ET-1. After the incubation, the reactions were stopped with 3.0 ml cold buffer containing 50 mM Tris and 10 mM $MgCl_2$, pH 7.5. Membrane bound radioactivity was separated from free ligand by filtering through Whatman GF/C filter paper and washing the filters 5 times with 3 ml of cold buffer using a Brandel cell harvester. Filter papers were counted in a gamma counter with an efficiency of 75%. $IC_{50}$'s for the compounds of this invention range from 0.1 nm to 50 µm.

II. In Vitro Vascular Smooth Muscle Activity

Rat aorta are cleaned of connective tissue and adherent fat, and cut into ring segments approximately 3 to 4 mm in length. Vascular rings are suspended in organ bath chambers (10 ml) containing Krebs-bicarbonate solution of the following composition (millimolar): NaCl, 112.0; KCl, 4.7; $KH_2PO_4$, 1.2; $MgSO_4$, 1.2; $CaCl_2$, 2.5; $NaHCO_3$, 25.0; and dextrose, 11.0. Tissue bath solutions are maintained at 37°C and aerated continuously with 95% $O_2$/ 5% $CO_2$. Resting tensions of aorta are maintained at 1 g and allowed to equilibrate for 2 hrs., during which time the bathing solution is changed every 15 to 20 min. Isometric tensions are recorded on Beckman R-611 dynographs with Grass FT03 force-displacement transducer. Cumulative concentration-response curves to ET-1 or other contractile agonists are constructed by the method of step-wise addition of the agonist. ET-1 concentrations are increased only after the previous concentration produces a steady-state contractile response. Only one concentration-response curve to ET-1 is generated in each tissue. ET receptor antagonists are added to paired tissues 30 min prior to the initiation of the concentration-response to contractile agonists.

ET-1 induced vascular contractions are expressed as a percentage of the response elicited by 60 mM KCl for each individual tissue which is determined at the beginning of each experiment. Data are expressed as the mean ± S.E.M. Dissociation constants ($K_b$) of competitive antagonists were determined by the standard method of Arunlakshana and Schild. The potency range for compounds of this invention range from 0.1 nM to 50 µm.

The following examples are illustrative and are not limiting of the compounds of this invention.

EXAMPLE 1

(1RS,2RS,3SR)-1-(4-Methoxyphenyl)-3-phenylindane-2-carboxylic acid

a) Ethyl (1RS)[1-hydroxy-1-(4-methoxyphenyl)]-3-phenylindene-2-carboxylate.

To dry magnesium turnings (0.88 g, 36 mmol) under an argon atmosphere was added, portionwise, a solution of p-bromoanisole (4.5 ml, 36 mmol) in 5% THF/ $Et_2O$ (37 ml). The resulting p-methoxyphenyl magnesium bromide solution was added to a solution of ethyl 1-oxo-3-phenylindene-2-carboxylate (5.0 g, 18 mmol) in $Et_2O$ (300 ml) under an argon atmosphere at 0°C. The resulting mixture was allowed to warm to room temperature and was stirred for 10 min. The mixture was partitioned between 3M HCl (100 ml) and EtOAc (200 ml). The organic extract was washed successively with $H_2O$, aqueous $NaHCO_3$, $H_2O$ and saturated aqueous NaCl and dried ($Na_2SO_4$). The solvent was removed *in vacuo* to provide a yellow oil which was treated with $Et_2O$/hexanes. The solid which formed was collected by filtration (3.47 g). The filtrate was concentrated under reduced pressure and purified by flash chromatography. The material which was isolated was treated with $Et_2O$/hexanes, and the additional solid which formed (1.76 g, 75% total yield) was collected by filtration to afford the title compound.

b) Ethyl (RS)-1-(4-methoxyphenyl)-3-phenylindene-2-carboxylate.

To a solution of ethyl (1RS) [1-hydroxy-1-(4-methoxyphenyl)]-3-phenylindene-2-carboxylate (4.65 g, 12.0 mmol) in $CH_2Cl_2$ (40 ml) at 0°C under an argon atmosphere was added triethylsilane (2.34 ml, 14.6 mmol), followed by boron trifluoride etherate (8.8 ml, 71 mmol). The reaction mixture was allowed to warm to room temperature and stirred for 10 min, at which time was added slowly 3M HCl (50 ml). The mixture was extracted with EtOAc (150 ml). The organic extract was washed successively with $H_2O$, aqueous $NaHCO_3$, $H_2O$ and saturated aqueous NaCl and dried. The solvent was removed *in vacuo*, and the residue was purified by flash chromatography on silica gel, eluting with 10% EtOAc/ hexanes to provide the title compound (4.2 g, 95%) as a mixture of Δ1 and Δ2 double bond isomers.

c) Ethyl (1RS,2SR,3SR)-1-(4-methoxyphenyl)-3-phenylindane-2-carboxylate.

To a solution of ethyl (RS)-1-(4-methoxyphenyl)-3-phenylindene-2-carboxylate (5.75 g, 15 mmol) in EtOAc (150 ml) was added 5% palladium on activated carbon (600 mg). The resulting suspension was stirred under an atmosphere of $H_2$ for 1 d, then was filtered through a pad of Celite. The filtrate was concentrated under reduced pressure to afford the title compound, which was used without further purification.

d) (1RS,2RS,3SR)-1-(4-Methoxyphenyl)-3-phenylindane-2-carboxylic acid.

To a solution of ethyl (1RS,2SR,3SR)-1-(4-methoxyphenyl)-3-phenylindane-2-carboxylate, (5.5 g, 14.8 mmol) in EtOH (70 ml) was added 5M NaOH (9 ml, 45 mmol). The resulting mixture was stirred under an argon atmosphere for 1 d, at which time $H_2O$ (70 ml) was added. The mixture was concentrated under reduced pressure. The aqueous

residue was extracted with $Et_2O$, and the $Et_2O$ extracts were discarded. The aqueous phase was acidified with 6M HCl and extracted several times with EtOAc. The combined EtOAc extracts were washed successively with H20 and saturated aqueous NaCl and dried. The solvent was removed *in vacuo* to provide an oily residue which crystallized upon standing. The solid material was recrystallized from EtOAc/hexanes to afford the title compound (4.25 g, 83%); m.p. 164 - 166°C.
$^1$H NMR ($CDCl_3$): δ 7.35 - 7.18 (m, 9H); 6.92 - 6.88 (m, 4H); 4.68 (d, 1H, J = 10 Hz); 4.64 (d, 1H, J = 10 Hz); 3.81 (s, 3H); 3.34 (t, 1H, J = 10 Hz).
MS : 345 [$(M+H)^+$].
Anal. Calc. for $C_{23}H_{20}O_3$: C, 80.21; H, 5.85.
Found C, 80.21; H 6.03.

EXAMPLE 2

(trans, trans)-1,3-Di(4-methoxyphenyl)indane-2-carboxylic acid

a) Ethyl 2-benzoyl-3-(4-hydroxyphenyl)propenoate.

To a solution of 4-hydroxybenzaldehyde (31.7 g, 0.26 mol) and ethyl benzoylacetate (45.5 ml, 0.26 mol) in EtOH (45 ml) under an argon atmosphere was added piperidine (2.6 ml, 0.026 mol) and acetic acid (3 drops). After stirring at room temperature overnight, the resulting solid mixture was treated with hot EtOH (700 ml), and then allowed to cool. The crystals which formed were collected by filtration to afford the title compound (61.0 g, 79%).

b) Ethyl (2RS,3SR)-3-(4-hvdroxyphenyl)-1-oxoindane-2-arboxylate.

To a mixture of ethyl 2-benzoyl-3-(4-hydroxyphenyl)propenoate (0.50 g, 1.7 mmol) in $CH_2Cl_2$ (15 ml) at 0°C under an argon atmosphere was added titanium tetrachloride (0.93 ml, 8.3 mmol). The resulting mixture was allowed to stir at room temperature overnight. The reaction was slowly quenched with 3M HCl, then partitioned between EtOAc (50 ml) and 3M HCl. The aqueous phase was extracted with EtOAc, and the combined organic extracts were washed successively with $H_2O$ and saturated aqueous NaCl, and dried ($Na_2SO_4$). The solvent was removed *in vacuo*, and the solid residue was recrystallized from EtOAc/hexanes to afford the title compound (410 mg, 82%).

c) Ethyl (2RS,3SR)-3-(4-t-butyldimethylsiloxyphenyl)-1-oxoindane-2-carboxylate.

To a solution of ethyl (2RS,3SR)-3-(4-hydroxyphenyl)-1-oxoindane-2-carboxylate (3.0 g, 10.2 mmol) in DMF (10 ml) under an argon atmosphere were added imidazole (1.72 g, 25.3 mmol) and t-butyldimethylchloro-silane (1.82 g, 12.1 mmol). The resulting mixture was allowed to stir at room temperature for 3 d, then was poured into dilute aqueous HCl and extracted with EtOAc (2x). The combined organic extracts were washed successively with $H_2O$, aqueous $NaHCO_3$, $H_2O$ and saturated aqueous NaCl and dried. The solvent was removed *in vacuo* to provide the title compound (5.40 g) which was used without further purification.

d) Ethyl 3-(4-t-Butyldimethylsiloxyphenyl)-1-oxoindene-2-carboxylate.

To a solution of ethyl (2RS,3SR)-3-(4-t-butyldimethylsiloxyphenyl)-1-oxoindane-2-carboxylate (130 mg, 0.32 mmol) in $CH_2Cl_2$ (3 ml) under an argon atmosphere was added 2,3-dichloro-5,6-dicyano-1,4-benzoquinone (80 mg, 0.35 mmol). The resulting mixture was stirred for 2.5 h. Aqueous $NaHSO_3$ and EtOAc were added, and the mixture was stirred for 5 min. The aqueous phase was separated and extracted with EtOAc, and the combined organic extracts were washed successively with aqueous $NaHCO_3$, $H_2O$ and saturated aqueous NaCl and dried. The solvent was removed *in vacuo*, and the residue was purified by flash chromatography on silica gel to afford the title compound (110 mg, 85%).

e) Ethyl (1RS)-3-(4-t-butyldimethylsiloxyphenyl)-1-hydroxy-1-(4-methoxyphenyl)indene-2-carboxylate.

To dry magnesium turnings (119 mg, 4.9 mmol) under an argon atmosphere was added, portionwise, a solution of p-bromoanisole (0.61 ml, 4.9 mmol) in 9:1 $Et_2O$/ THF (10 ml). The resulting p-methoxyphenyl magnesium bromide solution was added to a solution of ethyl 3-(4-t-butyldimethylsiloxyphenyl)-1-oxoindene-2-carboxylate (1.00 g, 2.5 mmol) in $Et_2O$ (60 ml) under an argon atmosphere at 0°C. The resulting mixture was allowed to warm to room temperature and was stirred for 5 min. The mixture was partitioned between 3M HCl and EtOAc. The organic extract was washed successively with $H_2O$, aqueous $NaHCO_3$, $H_2O$ and saturated aqueous NaCl and dried. The solvent was

removed *in vacuo* to provide the title compound (1.47 g) which was used without further purification.

f) Ethyl (RS)-1-(4-t-butyldimethylsiloxyphenyl)-3-(4-methoxyphenyl)indene-2-carboxylate.

To a solution of ethyl (1RS)-3-(4-t-butyldimethylsiloxyphenyl)-1-hydroxy-1-(4-methoxyphenyl)indene-2-carboxylate (2.5 mmol, prepared above) in $CH_2Cl_2$ (10 ml) at 0°C under an argon atmosphere was added triethylsilane (0.48 ml, 3.0 mmol), followed by boron trifluoride etherate (1.8 ml, 14.6 mmol). The reaction mixture was allowed to warm to room temperature and stirred for 10 min, at which time was added slowly 3M HCl. The mixture was extracted with EtOAc. The organic extract was washed successively with $H_2O$, aqueous $NaHCO_3$, $H_2O$ and saturated aqueous NaCl and dried. The solvent was removed *in vacuo,* and the residue was purified by flash chromatography on silica gel, eluting with 15% $Et_2O$/hexanes to provide the title compound as a mixture of Δ1 and Δ2 double bond isomers (820 mg, 67% for two steps).

g) Ethyl (1RS,2SR,3SR)-1-(4-t-butyldimethylsiloxyphenyl)-3-(4-methoxyphenyl)indane-2-carboxylate.

To a solution of ethyl (RS)-3-(4-t-butyldimethylsiloxyphenyl)-1-(4-methoxyphenyl)indene-2-carboxylate (mixture of Δ1 and Δ2 double bond isomers) (750 mg, 1.5 mmol) in EtOH (25 ml) was added 5% palladium on activated carbon (70 mg). The resulting suspension was stirred under an atmosphere of $H_2$ for 18 h, then was filtered through a pad of Celite. The filtrate was concentrated under reduced pressure to afford the title compound (730 mg, 97%), which was used without further purification.

h) Ethyl (1RS,2RS,3SR)-1-(4-hydroxyphenyl)-3-(4-methoxyphenyl)indane-2-carboxylate.

To a solution of ethyl (1RS,2SR,3SR)-1-(4-t-butyldimethylsiloxyphenyl)-3-(4-methoxyphenyl)indane-2-carboxylate (723 mg, 1.4 mmol) in EtOH (20 ml) was added 1M NaOH (1.6 ml, 1.6 mmol), and the resulting mixture was stirred at room temperature for 30 min. The mixture was then partitioned between 3M HCl and EtOAc. The aqueous phase was extracted with EtOAc, and the combined organic extracts were washed successively with $H_2O$ and saturated aqueous NaCl and dried. The solvent was removed *in vacuo* to afford the title compound (554 mg, 100%).

i) Ethyl (cis, cis)-1,3-di(4-methoxyphenyl)indane-2-carboxylate.

To a solution of ethyl (1RS,2RS,3SR)-1-(4-hydroxyphenyl)-3-(4-methoxyphenyl)indane-2-carboxylate (270 mg, 0.7 mmol) in acetonitrile (5 ml) at 0°C was added 1,8-diazabicyclo[5.4.0]undec-7-ene (0.25 ml, 1.7 mmol), followed by methyl iodide (0.5 ml, 8.0 mmol). The resulting mixture was allowed to warm to room temperature and was stirred overnight. The mixture was partitioned between EtOAc and dilute aqueous HCl. The organic extract was washed with saturated aqueous NaCl and dried. The solvent was removed *in vacuo,* and the residue was purified by flash chromatography to afford the title compound (40 mg, 32% based on recovered starting material).

j) (trans, trans)-1,3-Di (4-methoxyphenyl)indane-2-carboxylic acid.

To a solution of ethyl (cis, cis)-1,3-di(4-methoxyphenyl)indane-2-carboxylate (35 mg, 0.09 mmol) in EtOH (3 ml) was added 1M NaOH (0.25 ml, 0.25 mmol), and the resulting mixture was allowed to stir at room temperature overnight. Thin layer chromatographic analysis at this time indicated that the reaction was incomplete, so 5M NaOH (0.15 ml, 0.75 mmol) was added, and the mixture was allowed to stand at 0°C for 5 days. Water was added, and the mixture was concentrated under reduced pressure. The aqueous residue was extracted with $Et_2O$ (2x), and the $Et_2O$ extracts were discarded. The aqueous phase was acidified with 6M HCl and extracted several times with EtOAc. The combined EtOAc extracts were washed successively with $H_2O$ and saturated aqueous NaCl and dried. The solvent was removed *in vacuo* to provide an oily residue which crystallized upon standing. The solid material was recrystallized from EtOAc/ hexanes to afford the title compound (19 mg, 59%); m.p. 192 - 193°C.
$^1$H NMR (acetone-$d_6$) : δ 7.25 (dd, 4H, *J* = 6.6 Hz, 2.1 Hz); 7.21 - 7.18 (m, 2H); 6.92 (dd, 4H, *J* = 6.6 Hz, 2.1 Hz); 6.86 - 6.83 (m, 2H); 4.59 (d, 2H, *J* = 10 Hz); 3.79 (s, 6H); 3.26 (t, 1H, *J* = 10 Hz).
MS : 392 [$(M+NH_4)^+$].
Anal. Calc. for $C_{24}H_{22}O_4$: C, 76.99; H, 5.92.
Found C, 76.74; H 6.15.

EXAMPLE 3

(1RS,2SR,3SR)-1-(4-Methoxyphenyl)-3-(3,4-methylenedioxyphenyl)indane-2-carboxylic acid

a) 2-(3,4-Methylenedioxybenzoyl)benzoic acid.

To a solution of 2-bromobenzoic acid (12 g, 0.06 mol) in THF (200 ml) at -100°C under an argon atmosphere was added dropwise n-butyl lithium (50 ml of 2.5M solution in hexanes, 0.125 mol), maintaining the temperature below -90°C. Upon completion of the addition, the resulting solution was stirred at -100°C for 1 h, at which time was added slowly a solution of piperonylic acid chloride (11 g, 0.06 mol) in THF (50 ml), maintaining the temperature below -90°C. The resulting mixture was allowed to warm to -80°C and stirred for 1 h, then was allowed to slowly warm to room temperature and left to stand for 48 h. The reaction mixture was concentrated under reduced pressure, and the residue was partitioned between $Et_2O$ and 1M HCl. The organic phase was extracted with 10% aqueous NaOH. The NaOH extract was acidified with concentrated HCl, and the combined aqueous material was extracted with $Et_2O$. The $Et_2O$ extract was dried ($MgSO_4$) and concentrated under reduced pressure. The residue was purified by flash chromatography on silica gel, eluting with a solvent gradient of 10 - 30% EtOAc/0.1% HOAc/hexanes to afford the title compound as an off-white solid (4.5 g, 28%).

b) Diethyl 2-[2-(3,4-methylenedioxybenzoyl)benzoyl-malonate.

A solution of 2-(3,4-methylenedioxybenzoyl)benzoic acid (4.0 g, 14.8 mmol) in thionyl chloride (30 ml) was heated at reflux for 2 h, then allowed to cool and was concentrated under reduced pressure. The residue was dissolved in $Et_2O$ (50 ml) and to this was added a solution of diethyl magnesium malonate [prepared by the method of Walker and Hauser, JACS, *68,* 1386 (1946) using magnesium (0.8 g, 33.3 mmol) and diethyl malonate (4.9 g, 30.6 mmol)] in $Et_2O$. The resulting mixture was heated at reflux for 1 h, then allowed to cool and was poured into ice-cold 10% aqueous $H_2SO_4$ (100 ml). The aqueous phase was extracted with $Et_2O$, and the combined organic material was washed with saturated aqueous NaCl and dried. The solvent was removed under reduced pressure to afford the title compound as an orange oil, which was used without further purification.

c) Ethyl 3-(3,4-methylenedioxyphenyl)-1-oxoindene-2-carboxylate.

A solution containing diethyl 2-[2-(3,4-methylenedioxybenzoyl)benzoylmalonate (crude material prepared above) in 5% aqueous $Na_2CO_3$ (100 ml) was heated at reflux for 10 min. The reaction mixture was then allowed to cool, and the aqueous material was removed by decantation. The residue was placed in $H_2O$ (50 ml), and the mixture was heated at reflux, cooled and concentrated under reduced pressure. The residue was recrystallized from hexanes to afford the title compound as a yellow solid (5.0 g, 100% for two steps).

d) Ethyl (1RS)-1-hydroxy-1-(4-methoxyphenyl)-3-(3,4-methylenedioxyphenyl)indene-2-carboxylate.

A solution of 4-bromoanisole (0.89 g, 5.0 mmol) in 9 : 1 $Et_2O$/ THF (10 ml) was added to magnesium turnings (0.105 g, 5.0 mmol), and the resulting mixture was allowed to stir for 30 min. The resultant 4-methoxyphenyl magnesium bromide was added dropwise to a solution of ethyl 3-(3,4-methylenedioxyphenyl)-1-oxoindene-2-carboxylate (0.77 g, 2.4 mmol) in 10 : 1 $Et_2O$/ THF (55 ml) at 0°C. The resulting mixture was stirred at 0°C for 1 h and was then partitioned between EtOAc and 1M HCl. The aqueous phase was extracted with EtOAc, and the combined organic extracts were washed successively with 5% aqueous $NaHCO_3$ and saturated aqueous NaCl and dried ($MgSO_4$). The solvent was removed under reduced pressure, and the residue was purified by flash chromatography on silica gel, eluting with 10% EtOAc/hexanes to afford the title compound as a yellow glassy solid (0.80 g, 80%).

e) Ethyl (RS)-1-(4-methoxyphenyl)-3-(3,4-methylenedioxyphenyl)indene-2-carboxylate.

To a solution of ethyl (1RS)-1-hydroxy-1-(4-methoxyphenyl)-3-(3,4-methylenedioxyphenyl)indene-2-carboxylate (0.80 g, 1.9 mmol) in $CH_2Cl_2$ (10 ml) at 0°C under an argon atmosphere was added triethylsilane (0.28 g, 2.4 mmol), followed by boron trifluoride etherate (1 ml, 8.1 mmol). The resulting solution was stirred at 0°C for 10 min, and was then partitioned between EtOAc and 3M HCl. The organic extract was washed with saturated aqueous NaCl and dried ($MgSO_4$). The solvent was removed *in vacuo,* and the residue was filtered through a pad of silica gel, eluting with $CH_2Cl_2$. The title compound (mixture of Δ1 and Δ2 double bond isomers) was obtained as a glassy, yellow solid (0.72 g, 94%).

f) Ethyl(1RS,2RS,3SR)-1-(4-methoxyphenyl)-3-(3,4-methylenedioxyphenyl)indane-2-carboxylate.

To a solution of ethyl (RS)-1-(4-methoxyphenyl)-3-(3,4-methylenedioxyphenyl)indene-2-carboxylate (0.72 g, 1.7 mmol) in EtOH (30 ml) was added 10% palladium on activated carbon (1 g). The resulting suspension was stirred under an atmosphere of $H_2$ for 56 h and filtered. The filtrate was concentrated under reduced pressure to afford the title compound as a yellow solid (0.70 g, 95%), which was used without further purification.

g) (1RS,2SR,3SR)-1-(4-methoxyphenyl)-3-(3,4-methylenedioxyphenyl)indane-2-carboxylic acid.

To a solution of ethyl (1RS,2RS,3SR)-1-(4-methoxyphenyl)-3-(3,4-methylenedioxyphenyl)indane-2-carboxylate (0.10 g, 0.2 mmol) in EtOH (5 ml) was added a solution of sodium hydroxide (0.10 g, 2.5 mmol) in $H_2O$ (2 ml). The resulting mixture was stirred at room temperature overnight. The mixture was acidified, and the solid which formed was collected by filtration and dried under reduced pressure to afford the title compound as a tan solid (0.04 g, 86%). $^1$H NMR ($CDCl_3$) : δ 7.25 (m, 5H) ; 6.90 (m, 4H) ; 6.77 (d, 2H, *J* = 7 Hz); 5.95 (m, 2H); 4.61 (d, 2H, *J* = 10 Hz); 3.81 (s, 3H); 3.25 (t, 2H, *J* = 10 Hz). MS: 387 [(M-H$^+$]. Anal. Calc. for $C_{24}H_{20}O_5$·□ $H_2O$: C, 73.79; H, 5.22. Found C, 76.73; H 5.21.

EXAMPLE 4

(1RS, 2SR, 3SR)-1-(4-Fluorophenyl)-3-(3,4-methylenedioxyphenyl)indane-2-carboxylic acid

a) Ethyl (1RS)-1-(4-fluorophenyl)-1-hydroxy-3-(3,4-methylenedioxyphenyl)indene-2-carboxylate.

To a solution of ethyl 3-(3,4-methylenedioxyphenyl)-1-oxoindene-2-carboxylate (100 mg, 0.31 mmol) in THF (5 ml) under an argon atmosphere at 0°C was added a solution of freshly prepared 4-fluorophenyl magnesium bromide (0.62 mmol). After stirring for 45 min, the mixture was partitioned between 3M HCl and EtOAc. The organic extract was washed successively with $H_2O$, 5% aqueous $NaHCO_3$ and saturated aqueous NaCl. The solvent was removed *in vacuo,* and the residue was purified by flash chromatography, eluting with 15% EtOAc/ hexanes to afford the title compound (45 mg, 35%).

b) Ethyl (RS)-1-(4-fluorophenyl)-3-(3,4-methylenedioxyphenyl)indene-2-carboxylate.

To a solution of ethyl (1RS)-1-(4-fluorophenyl)-1-hydroxy-3-(3,4-methylenedioxyphenyl)indene-2-carboxylate (45 mg, 0.11 mmol) in $CH_2Cl_2$ (3 ml) at 0°C was added triethylsilane (38 μl, 0.24 mmol), followed by boron trifluoride etherate (121 μl, 0.98 mmol). The reaction mixture was allowed to warm to room temperature and stirred for 15 min, at which time was added slowly 3M HCl. The mixture was extracted with EtOAc. The organic extract was washed successively with $H_2O$, 5% aqueous $NaHCO_3$ and saturated aqueous NaCl. The solvent was removed *in vacuo* to provide the title compound (40 mg, 90%) as a mixture of Δ1 and Δ2 double bond isomers.

c) Ethyl (1RS, 2RS, 3SR)-1-(4-fluorophenyl)-3-(3,4-methylenedioxyphenyl)indane-2-carboxylate.

To a solution of ethyl (RS)-1-(4-fluorophenyl)-3-(3,4-methylenedioxyphenyl)indene-2-carboxylate (40 mg, 0.10 mmol) in EtOH (3 ml) was added 10% palladium on activated carbon (45 mg). The resulting suspension was stirred under an atmosphere of $H_2$ overnight, then was filtered through a pad of Celite. The filtrate was concentrated under reduced pressure to afford the title compound (40 mg, 100%), which was used without further purification.

d) (1RS, 2SR, 3SR)-1-(4-Fluorophenyl)-3-(3,4-methylenedioxyphenyl)indane-2-carboxylic acid.

To a solution of ethyl (1RS, 2RS, 3SR)-1-(4-fluorophenyl)-3-(3,4-methylenedioxyphenyl)indane-2-carboxylate (60 mg, 0.15 mmol) in EtOH (0.5 ml) was added 6M KOH (0.14 ml, 0.84 mmol). The resulting mixture was allowed to stir at room temperature overnight, then was concentrated under reduced pressure. The residue was partitioned between $H_2O$ and $Et_2O$. The aqueous phase was acidified with 3M HCl and extracted several times with EtOAc. The combined EtOAc extracts were washed successively with $H_2O$ and saturated aqueous NaCl and dried ($MgSO_4$). The solvent was removed *in vacuo* to afford an oil, which was crystallized from EtOAc/ hexanes. The title compound was obtained as an off-white crystalline solid (22 mg, 39%); m.p. 146 - 149°C.
$^1$H NMR ($CDCl_3$): δ 7.23 (m, 4H); 6.96 (m, 1H); 6.90 (m, 1H); 6.79 (s, 2H); 6.75 (s, 1H); 5.96 (m, 2H); 4.62 (apparent br t, 2H, *J* = 10 Hz); 3.25 (t, 1H, *J* = 10 Hz). MS m/e (rel. int.): 753 [(2M+1)$^+$, 3].
Anal. Calcd. for $C_{23}H_{17}FO_4$: C, 73.40; H, 4.55.
Found: C, 73.19; H, 4.45.

EXAMPLE 5

(1RS, 2SR, 3SR)-1-(3-Methoxyphenyl)-3-(3,4-methylenedioxyphenyl)indane-2-carboxylic acid a) Ethyl (1RS)-1-hydroxy-1-(3-methoxyphenyl)-3-(3,4-methylenedioxyphenyl)indene-2-carboxylate.

To a solution of ethyl 3-(3,4-methylenedioxyphenyl)-1-oxoindene-2-carboxylate (100 mg, 0.31 mmol) in THF (2 ml) under an argon atmosphere at 0°C was added a solution of freshly prepared 3-methoxyphenyl magnesium bromide (0.31 mmol). After stirring for 15 min, additional 3-methoxyphenyl magnesium bromide (0.06 mmol) was added. Stirring was continued for 45 min, at which time thin layer chromatographic analysis indicated that the reaction was incomplete. Additional 3-methoxyphenyl magnesium bromide (0.12 mmol) was added. After stirring for 2 h more, the mixture was partitioned between 3M HCl and EtOAc. The organic extract was washed successively with $H_2O$, 5% aqueous $NaHCO_3$, $H_2O$ and saturated aqueous NaCl. The solvent was removed *in vacuo*, and the residue was purified by flash chromatography, eluting with 15% EtOAc/hexanes to afford the title compound (150 mg, 100%).

b) Ethyl (1RS)-1-(3-methoxyphenyl)-3-(3,4-methylenedioxyphenyl)indene-2-carboxylate.

To a solution of ethyl (1RS)-1-hydroxy-1-(3-methoxyphenyl)-3-(3,4-methylenedioxyphenyl)-indene-2-carboxylate (150 mg, 0.35 mmol) in $CH_2Cl_2$ was added triethylsilane (67 µl, 0.42 mmol), followed by boron trifluoride etherate (213 µl, 1.73 mmol). The reaction mixture was allowed to stir for 30 min, at which time was added slowly 5% aqueous HCl. The mixture was extracted with EtOAc. The organic extract was washed successively with $H_2O$, 5% aqueous $NaHCO_3$, $H_2O$ and saturated aqueous NaCl and dried ($MgSO_4$). The solvent was removed *in vacuo*, and the residue was purified by flash chromatography, eluting with 10% EtOAc/hexanes to provide the title compound (45 mg, 31%) as a mixture of Δ1 and Δ2 double bond isomers.

c) Ethyl (RS, 2RS, 3SR)-1-(3-methoxyphenyl)-3-(3,4-methylenedioxyphenyl)indane-2-carboxylate.

To a solution of ethyl (RS)-1-(3-methoxyphenyl)-3-(3,4-methylenedioxyphenyl)indene-2-carboxylate (45 mg, 0.11 mmol) in EtOH (3 ml) was added 10% palladium on activated carbon (45 mg). The resulting suspension was shaken on a Parr hydrogenator at 50 psi $H_2$ overnight, then was filtered through a pad of Celite. The filtrate was concentrated under reduced pressure to afford the title compound (43 mg, 94%), which was used without further purification.

d) (1RS, 2SR, 3SR)-1-(3-Methoxyphenyl)-3-(3,4-methylenedioxyphenyl)indane-2-carboxylic acid.

To a solution of ethyl (1RS, 2RS, 3SR)-1-(3-methoxyphenyl)-3-(3,4-methylenedioxyphenyl)indane-2-carboxylate (43 mg, 0.10 mmol) in EtOH (1 ml) was added 6M KOH (0.10 mL, 0.60 mmol). The resulting mixture was allowed to stir at room temperature overnight, then was partitioned between $H_2O$ and $Et_2O$. The aqueous phase was acidified with 3M HCl and extracted several times with EtOAc. The combined EtOAc extracts were washed successively with $H_2O$ and saturated aqueous NaCl and dried ($MgSO_4$). The solvent was removed *in vacuo* to afford an oil, which was crystallized from $Et_2O$/ hexanes. The title compound was obtained as a solid; m.p. 131 - 133°C.
$^1$H NMR ($CDCl_3$): δ 7.21 (m, 3H); 6.97 - 6.73 (m, 8H); 5.95 (m, 2H); 4.61 (apparent br t, 2H, *J* = 9 Hz); 3.67 (s, 3H); 3.30 (t, 1H, *J* = 9 Hz).
MS m/e (rel. int.): 777 [$(2M+1)^+$, 65].
Anal. Calcd. for $C_{24}H_{20}O_5$: C, 74.21; H, 5.19.
Found: C, 74.71; H, 5.47.

EXAMPLE 6

(1RS, 3RS)-1,3-Di-(3,4-methylenedioxyphenyl)indane-2-carboxylic acid

a) Ethyl (1RS)-1,3-di-(3,4-methylenedioxyphenyl)-1-hydroxyindene-2-carboxylate.

To dry magnesium turnings (0.25 g, 10 mmol) under an argon atmosphere was added a solution of 4-bromo-1,2-methylenedioxybenzene (2.1 g, 10 mmol) in 1:10 THF/$Et_2O$ (22 ml). The resulting solution was allowed to stir at room temperature for 2 h. During this time, additional THF (4 ml) was added. The resulting 3,4-methylenedioxyphenylmagnesium bromide was added to a solution of ethyl 3-(3,4-methylenedioxyphenyl)-1-oxoindene-2-carboxylate (0.50 g, 2 mmol) in 1:4 THF/$Et_2O$ (25 ml) under an argon atmosphere at 0°C. The resulting mixture was stirred at 0°C for 15 min, at which time 1M HCl (50 ml) was added. The phases were separated and the aqueous phase was extracted

with $Et_2O$. The combined organic extracts were washed with saturated aqueous NaCl and dried ($MgSO_4$). The solvent was removed *in vacuo*, and the residue was purified by flash chromatography, eluting with 10% EtOAc/hexanes to afford the title compound as a yellow solid (0.29 g, 42%).

b) Ethyl (RS)-1,3-Di-(3,4-methylenedioxyphenyl)indene-2-carboxylate.

To a solution of ethyl (1RS)-1,3-di-(3,4-methylenedioxyphenyl)-1-hydroxyindene-2-carboxylate (0.29 g, 0.65 mmol) in $CH_2Cl_2$ (3 ml) at 0°C under an argon atmosphere was added triethylsilane (91 mg, 0.78 mmol), followed by boron trifluoride etherate (0.3 ml, 2.4 mmol). The reaction mixture was stirred for 10 min, at which time was added ice-cold 1M HCl, and the mixture was extracted with EtOAc. The organic extract was washed with saturated aqueous NaCl and dried ($MgSO_4$). The solvent was removed *in vacuo,* and the residue was placed on a small pad of silica gel, eluting with $CH_2Cl_2$ to provide the title compound (257 mg, 92%).

c) Ethyl (1RS, 3RS)-1,3-di-(3,4-methylenedioxyphenyl)indane-2-carboxylate.

Ethyl (RS)-1,3-di-(3,4-methylenedioxyphenyl)indene-2-carboxylate (163 mg, 0.38 mmol) was placed in MeOH (0.05 ml), and to this was added $SmI_2$ (10 ml of 0.1M solution in THF, 1.0 mmol). The resulting mixture was stirred under an argon atmosphere overnight, at which time thin layer chromatographic analysis indicated that the reaction was incomplete. Additional $SmI_2$ (5ml of 0.1M solution in THF, 0.5 mmol) was added, and stirring was continued for 2 h. The reaction mixture was partitioned between $Et_2O$ and 5% aqueous $Na_2S_2O_3$. The organic extract was washed with saturated aqueous NaCl and dried ($MgSO_4$). The solvent was removed under reduced pressure, and the residue was purified by flash chromatography, eluting with 10% EtOAc/ hexanes to afford the title compound as a colorless, glassy solid (120 mg, 75%).

d) (1RS, 3RS)-1,3-Di-(3,4-methylenedioxyphenyl)indane-2-carboxylic acid.

To a solution of ethyl (1RS, 3RS)-1,3-di-(3,4-methylenedioxyphenyl)indane-2-carboxylate (75 mg, 0.17 mmol) in EtOH (20 ml) was added NaOH (0.10 g, 2.5 mmol). The resulting mixture was allowed to stir at room temperature for 3 d, at which time thin layer chromatographic analysis indicated that the reaction was incomplete. The mixture was then heated at reflux for 36 h, allowed to cool and was concentrated under reduced pressure. To the residue was added concentrated HCl, and the solid which formed was collected by filtration and dried. The solid was triturated with boiling hexanes to afford the title compound as a white solid (50 mg, 73%); m.p. 182 - 185°C.
$^1$H NMR ($CDCl_3$) : δ 7.25 (m, 2H); 7.15 (m, 1H); 7.00 (m, 1H); 6.76 (s, 2H); 6.68 (m, 2H); 6.50 (dd, 1H, *J* = 8, 1 Hz); 6.40 (d, 1H, *J*= 2 Hz); 5.94 (s, 2H); 5.90 (d, 1H, *J* = 1 Hz); 5.87 (d, 1H, *J* = 1 Hz); 4.84 (d, 1H, *J* = 10 Hz); 4.78 (d, 1H, *J* = 10 Hz); 3.63 (dd, 1H, *J* = 10 Hz, 9 Hz).
MS: 402 $(M)^+$.
Anal. Calcd. for $C_{24}H_{18}O_6 \cdot 1/5\ H_2O$: C, 71.00; H, 4.52. Found: C, 71.13; H, 4.46.

EXAMPLE 7

(trans, trans)-1,3-Di-(3,4-methylenedioxyphenyl)indane-2-carboxylic acid

a) Ethyl (cis, cis)-1,3-di-(3,4-methylenedioxyphenyl)-indane-2-carboxylate

To a solution of ethyl (RS)-1,3-di-(3,4-methylenedioxyphenyl)indene-2-carboxylate (93 mg, 0.22 mmol) in EtOH (2 ml) was added 10% palladium on activated carbon (0.10 g). The resulting suspension was shaken on a Parr hydrogenator at 55 psi $H_2$ for 2 d, then was filtered through a pad of Celite. The filtrate was concentrated under reduced pressure to afford the title compound (45 mg, 48%) as a glassy, yellow solid, which was used without further purification.

b) (trans, trans)-1,3-Di-(3,4-methylenedioxyphenyl)indane-2-carboxylic acid.

To a solution of ethyl (cis, cis)-1,3-di-(3,4-methylenedioxyphenyl)indane-2-carboxylate (45 mg, 0.1 mmol) in 2 : 1 EtOH/ $H_2O$ (15 ml) was added sodium hydroxide (50 mg, 1.2 mmol). The resulting solution was allowed to stir at room temperature overnight, then was concentrated under reduced pressure. The residue was treated with concentrated HCl, and the solid which formed was collected by filtration and dried. The solid was recrystallized from $Et_2O$/ hexanes to afford the title compound as a light tan solid (12 mg, 30%); m.p. 188 - 191°C.

EXAMPLE 8

(1RS, 2RS, 3SR)-1-(3,4-Methylenedioxyphenyl)-3-phenylindane-2-carboxylic acid

a) Ethyl (1RS)-1-hydroxy-1-(3,4-methylenedioxyphenyl)3-phenylindene-2-carboxylate.

To a solution of ethyl 1-oxo-3-phenylindene-2-carboxylate (1.0 g, 3.6 mmol) in THF (35 ml) under an argon atmosphere at 0°C was added a solution of freshly prepared 3,4-methylenedioxyphenyl magnesium bromide (5.4 mmol). After stirring for 30 min, the mixture was partitioned between 3M HCl and EtOAc. The organic extract was washed successively with $H_2O$, 5% aqueous $NaHCO_3$ and saturated aqueous NaCl and dried ($MgSO_4$). The solvent was removed *in vacuo*, and the residue was purified by flash chromatography, eluting with 10% EtOAc/hexanes to afford the title compound (1.03 g, 72%).

b) Ethyl (RS)-1-(3,4-Methylenedioxyphenyl)-3-phenylindene-2-carboxylate.

To a solution of ethyl (1RS)-1-hydroxy-1-(3,4-methylenedioxyphenyl)-3-phenylindene-2-carboxylate (1.03 g, 2.58 mmol) in $CH_2Cl_2$ (40 mL) was added triethylsilane (0.49 ml, 3.07 mmol), followed by boron trifluoride etherate (1.55 ml, 12.6 mmol). The reaction mixture was allowed to stir for 15 min, at which time was added slowly 3M HCl. The mixture was extracted with EtOAc. The organic extract was washed successively with $H_2O$, 5% aqueous $NaHCO_3$ and saturated aqueous NaCl. The solvent was removed *in vacuo* to provide the title compound (1.00 g, 100%) as a mixture of Δ1 and Δ2 double bond isomers.

c) Ethyl (1RS, 2SR, 3SR)-1-(3,4-methylenedioxyphenyl)-3-phenylindane-2-carboxylate.

To a solution of ethyl (RS)-1-(3,4-methylenedioxyphenyl)-3-phenylindene-2-carboxylate (1.00 g, 2.60 mmol) in EtOH (25 ml) was added 10% palladium on activated carbon (30 mg). The resulting suspension was stirred under an atmosphere of $H_2$ overnight. Thin layer chromatographic analysis indicated that the reaction was incomplete, so additional 10% palladium on activated carbon (30 mg) was added, and the mixture was shaken on a Parr hydrogenator at 30 psi $H_2$ for 2 d. At this time, thin layer chromatographic analysis again indicated that the reaction was incomplete. The reaction mixture was filtered through a pad of Celite, and 10% palladium on activated carbon (250 mg) was added. The reaction mixture was shaken on a Parr hydrogenator at 60 psi $H_2$ overnight. Filtration and repetition of the latter hydrogenation conditions led to complete consumption of starting material. The reaction mixture was filtered through a pad of Celite, and the filtrate was concentrated under reduced pressure to afford the title compound (650 mg, 65%), which was used without further purification.

d) (1RS, 2RS, 3SR)-1-(3,4-Methylenedioxyphenyl)-3-phenylindane-2-carboxylic acid.

To a solution of ethyl (1RS, 2SR, 3SR)-1-(3,4-methylenedioxyphenyl)-3-phenylindane-2-carboxylate (650 mg, 1.68 mmol) in EtOH containing a few drops of THF was added 6M KOH (1.68 ml, 10.1 mmol). The resulting mixture was allowed to stir at room temperature overnight, then was concentrated under reduced pressure. The residue was partitioned between $H_2O$ and $Et_2O$. The aqueous phase was acidified with 3M HCl and extracted several times with EtOAc. The combined EtOAc extracts were washed successively with $H_2O$ and saturated aqueous NaCl and dried ($MgSO_4$). The solvent was removed *in vacuo* to afford an oil, which was crystallized from EtOAc/hexanes. The title compound was obtained as a solid (305 mg, 51%); m.p. 186 - 187°C. Anal. Calcd. for $C_{23}H_{18}O_4$: C, 77.08; H, 5.06. Found: C, 76.60; H, 5.08.

EXAMPLE 9

(1RS, 2SR, 3SR)-1-(4-Methoxyphenyl)-3-(3,4-methylenedioxyphenyl)-2-(tetrazol-5-yl)indane

a) (1RS, 2SR, 3SR)-1-(4-Methoxyphenyl)-3-(3,4-methvlenedioxyphenyl)indane-2-carboxamide.

A mixture of (1RS, 2SR, 3SR)-1-(4-methoxyphenyl)-3-(3,4-methylenedioxyphenyl)indane-2-carboxylic acid (250 mg, 0.64 mmol) in $SOCl_2$ (2.5 ml) was allowed to stir overnight under an argon atmosphere. The reaction mixture was concentrated under reduced pressure, and the residue was dissolved in benzene (5 ml). To the resulting mixture under an argon atmosphere was added concentrated $NH_4OH$ (5 ml). The solid which formed was collected by filtration, washed with $H_2O$ and dried *in vacuo* to afford the title compound (185 mg, 75%).

b) (1RS, 2SR, 3SR)-1-(4-Methoxyphenyl)-3-(3,4-methylenedioxyphenyl)indane-2-carbonitrile.

To ice-cold DMF (1 ml) under an argon atmosphere was added oxalyl chloride (68µl, 0.78mmol). After stirring for 5 min at 0°C, a solution of (1RS, 2SR, 3SR)-1-(4-methoxyphenyl)-3-(3,4-methylenedioxyphenyl)indane-2-carboxamide (150 mg, 0.39 mmol) in DMF (2 ml) was added, and stirring was continued for an additional 10 min at 0°C. The reaction mixture was partitioned between EtOAc and 3M HCl. The aqueous phase was extracted with EtOAc, and the combined organic extracts were washed successively with $H_2O$, aqueous $NaHCO_3$, $H_2O$ and saturated aqueous NaCl and dried. The solvent was removed *in vacuo* to afford the title compound as a white solid (135 mg, 94%) which was used without further purification.

c) (1RS, 2SR, 3SR)-1-(4-Methoxyphenyl)-3-(3,4-methylenedioxyphenyl)-2-(tetrazol-5-yl)indane.

To THF (2.5 ml) at -78°C under an argon atmosphere was added aluminum chloride (90 mg, 0.67 mmol). After slowly warming to room temperature, sodium azide (130 mg, 2.2 mmol) was added, and the resulting mixture was heated at 70°C for 5 min, then cooled to room temperature. To the reaction mixture was added a solution of (1RS, 2SR, 3SR)-1-(4-methoxyphenyl)-3-(3,4-methylenedioxyphenyl)indane-2-carbonitrile (125 mg, 0.34 mmol) in THF (2.5 ml). After heating at 70°C overnight, thin layer chromatographic analysis of the reaction mixture indicated the presence of starting material, so additional $Al(N_3)_3$ was prepared as above (1.34 mmol) in THF. To this was added the reaction mixture, and heating at 70°C was resumed for an additional 5 h. The mixture was partitioned between EtOAc and 3M HCl. The aqueous phase was extracted with EtOAc, and the combined organic extracts were washed successively with $H_2O$ and saturated aqueous NaCl and dried. The solvent was removed *in vacuo*, and the residue was crystallized from EtOAc/hexanes to afford the title compound (78 mg, 56%). A portion of this material was further purified by MPLC (LiChroprep RP-18, MeOH/$H_2O$=60/40) and then recrystallized; m.p. 155 - 157°C (EtOAc/ hexanes).
$^1$H NMR ($CDCl_3$): δ 7.28 - 7.15 (m, 4H); 7.03 - 6.95 (m, 2H); 6.87 - 6.84 (m, 2H); 6.74 (s, 3H); 5.94 (d, 1H, *J* = 1.2 Hz); 5.92 (d, 1H, *J* = 1.2 Hz); 4.79 (d, 1H, *J* = 11.6 Hz); 4.73 (d, 1H, *J* = 11.6 Hz); 3.79 (S, 3H); 3.65 (t, 1H, *J* = 11.6 Hz).
MS (m/e) : 413.2 [$(M+H)^+$].

EXAMPLE 10

(1RS, 2SR, 3RS)-1-(2-Methoxyphenyl)-3-(3,4-methylenedioxyphenyl)indane-2-carboxylic acid

a) Ethyl (1RS)-1-hydroxy-1-(2-methoxyphenyl)-3-(3,4-methylenedioxyphenyl)indene-2-carboxylate.

To dry magnesium turnings (81 mg, 3.4 mmol) under an argon atmosphere was added a solution of 2-bromoanisole (0.64 g, 3.4 mmol) in 5 : 1 THF/ $Et_2O$ (3 ml). A portion of the resulting 2-methoxyphenyl magnesium bromide solution (0.45 ml, 0.51 mmol) was added dropwise to a solution of ethyl 3-(3,4-methylenedioxyphenyl)-1-oxoindene-2-carboxylate (100 mg, 0.34 mmol) in THF (6 ml) under an argon atmosphere at 0°C. After stirring for 15 min, the mixture was partitioned between 3M HCl and EtOAc. The organic extract was washed successively with $H_2O$, 5% aqueous $NaHCO_3$, $H_2O$ and saturated aqueous NaCl. The solvent was removed *in vacuo,* and the residue was purified by flash chromatography, eluting with 15% EtOAc/ hexanes to afford the title compound. (100 mg, 68%).

b) Ethyl (RS)-1-(2-methoxyphenyl)-3-(3,4-methylenedioxyphenyl)indene-2-carboxylate.

To a solution of ethyl (1RS)-1-hydroxy-1-(2-methoxyphenyl)-3-(3,4-methylenedioxyphenyl)indene-2-carboxylate (100 mg, 0.23 mmol) in $CH_2Cl_2$ (5 ml) was added triethylsilane (32 mg, 0.28 mmol), followed by boron trifluoride etherate (0.13 ml, 1.05 mmol). The reaction mixture was allowed to warm to room temperature and stirred for 10 min, at which time was added slowly 3M HCl. The mixture was extracted with EtOAc. The organic extract was washed successively with $H_2O$, 5% aqueous $NaHCO_3$, $H_2O$ and saturated aqueous NaCl and dried ($MgSO_4$). The solvent was removed *in vacuo* to provide the title compound (91 mg, 96%) as a mixture of Δ1 and Δ2 double bond isomers.

c) Ethyl (1RS, 2RS, 3RS)-1-(2-methoxyphenyl)-3-(3,4-methylenedioxyphenyl)indane-2-carboxylate

To a solution of ethyl (RS)-1-(2-methoxyphenyl)-3-(3,4-methylenedioxyphenyl)indene-2-carboxylate (90 mg, 0.22 mmol) in EtOH (10 ml) was added 10% palladium on activated carbon (90 mg). The resulting suspension was shaken on a Parr hydrogenator at 60 psi $H_2$ overnight, then was filtered through a pad of Celite. The filtrate was concentrated under reduced pressure to afford the title compound (90 mg, 100%), which was used without further purification.

d) (1RS, 2SR, 3RS)-1-(2-Methoxyphenyl)-3-(3,4-methylenedioxyphenyl)indane-2-carboxylic acid.

To a solution of ethyl (1RS, 2RS, 3RS)-1-(2-methoxyphenyl)-3-(3,4-methylenedioxyphenyl)indane-2-carboxylate (90 mg, 0.22 mmol) in EtOH (2 ml) containing a few drops of THF was added 6M KOH (0.22 ml, 1.32 mmol). The resulting mixture was allowed to stir at room temperature overnight, then was concentrated under reduced pressure. The residue was partitioned between $H_2O$ and $Et_2O$. The aqueous phase was acidified with 3M HCl and extracted with EtOAc. The EtOAc extract was washed successively with $H_2O$ and saturated aqueous NaCl and dried ($MgSO_4$). The solvent was removed *in vacuo* to afford the title compound (40 mg, 49%). $^1$H NMR ($CDCl_3$): δ 7.37 - 6.73 (m, 11H); 5.93 (m, 2H); 5.03 (d, 1H, *J* = 10 Hz); 4.67 (d, 1H, *J* = 10 Hz); 3.70 (s, 3H); 3.38 (t, 1H, *J* = 10 Hz).

EXAMPLE 11

(1RS, 2SR, 3SR)-5-Hydroxy-3-(4-methoxyphenyl)-1-(3,4-methyenedioxyphenyl)indane-2-carboxylic acid. sodium salt

a) 3-Benzyloxyacetophenone.

To a mixture of sodium hydride (4.5 g of 80% mineral oil dispersion, 0.15 mol), which had been washed free of mineral oil, in DMF (25 ml) was added, dropwise with cooling, a solution of 3-hydroxyacetophenone (20.5 g, 0.15 mol) in DMF (25 ml). Upon completion of the addition, the mixture was allowed to stir at room temperature for 15 min, at which time was added benzyl bromide (25.6 g, 0.15 mol). The resulting mixture was allowed to stir at room temperature overnight, then was partitioned between EtOAc and 3M HCl. The aqueous phase was extracted with EtOAc, and the combined organic extracts were washed successively with 1M NaOH, $H_2O$ and saturated aqueous NaCl and dried. The solvent was removed *in vacuo* to afford the title compound (33 g, 97%), which was used without further purification.

b) Methyl 2-(3-benzyloxy)benzoylacetate.

To a mixtureof sodium hydride (28.3 g of 80% mineral oil dispersion, 0.94 mol), which had been washed free of mineral oil, in dimethyl carbonate (100 ml) under an argon atmosphere was added, over 30 min, a solution of 3-benzyloxyacetophenone (92.3 g, 0.41 mol) in dimethyl carbonate (150 ml). Upon completion of the addition, the mixture was heated at reflux for 30 min, then was cooled in an ice bath and quenched by the slow addition of 3M HCl. The mixture was partitioned between EtOAc and 3M HCl, and the aqueous phase was extracted with EtOAc. The combined organic extracts were washed successively with $H_2O$, aqueous $NaHCO_3$, $H_2O$ and saturated aqueous NaCl and dried. The solvent was removed *in vacuo* to afford the title compound (112.5 g, 97%).

c) Methyl 2-(3-benzyloxybenzoyl)-3-(3,4-methylenedioxyphenyl)propenoate.

A mixture containing methyl 2-(3-benzyloxy)benzoylacetate (75.0 g, 0.26 mol), piperonal (43.6 g, 0.29 mol), acetic acid (3.6 ml) and piperidine (1.2 ml) in benzene (70 ml) was heated at reflux, with azeotropic removal of $H_2O$. After heating at reflux for 4 h, the reaction mixture was concentrated *in vacuo,* and the residue was crystallized from EtOH to afford the title compound (93.5 g, 85%); m.p. 116 - 118°C.

d) Methyl (1RS,2SR)-5-benzyloxy-1-(3,4-methylenedioxyphenyl)-3-oxoindane-2-carboxylate.

To trifluoroacetic acid (150 ml) at 0°C under an argon atmosphere was added methyl 2-(3-benzyloxybenzoyl)-3-(3,4-methylenedioxyphenyl)propenoate (80.0 g, 0.19 mol). The mixture was allowed to warm to room temperature and stirred for 30 min, at which time the mixture was concentrated under reduced pressure. The residue was dissolved in EtOAc and washed successively with aqueous $NaHCO_3$, $H_2O$ and saturated aqueous NaCl and dried. The solvent was removed *in vacuo,* and the oily residue was crystallized from EtOAc/hexanes to afford the title compound (51.3 g, 64%); m.p. 148 - 150°C.

e) Methyl 5-benzyloxy-1-(3,4-methylenedioxyphenyl)-3-oxoindene-2-carboxylate.

To a solution of methyl 5-benzyloxy-1-(3,4-methylenedioxyphenyl)-3-oxoindane-2-carboxylate (27.3 g, 65.6 mmol) in benzene (90 ml), cooled in an ice-$H_2O$ bath, was added 2,3-dichloro-5,6-dicyano-1,4-benzoquinone (15.4 g, 67.8 mmol). The resulting mixture was stirred at 0°C for 1 h, allowed to warm to room temperature for 1.5 h, and finally warmed to 40°C for 1 h. The solid which formed was removed by filtration and washed with benzene. The combined filtrate and washings were poured into EtOAc (200 ml) and washed successively with aqueous $Na_2CO_3$ (3x), $H_2O$ (3x), 3M HCl, $H_2O$ (3x) and saturated aqueous NaCl and dried. The solvent was removed *in vacuo,* and the residue was

crystallized from EtOAc/hexanes to afford the title compound (16.4 g, 60%) as a red crystalline solid; m.p. 140 - 141°C.

f) Methyl (3R5) -5-benzyloxy-3-hydroxy-3-(4-methoxyphenyl)-1-(3,4-methylenedioxyphenyl)indene-2-carboxylate.

To dry magnesium turnings (0.96 g, 40 mmol) under an argon atmosphere was added a solution of 4-bromoanisole (7.48 g, 40 mmol) in 9 : 1 $Et_2O$/THF (50 ml). The resulting 4-methoxyphenyl magnesium bromide solution was added portionwise to a solution of methyl 5-benzyloxy-1-(3,4-methylenedioxyphenyl)-3-oxoindene-2-carboxylate (8.29 g, 20 mmol) in THF (250 ml) under an argon atmosphere. Upon completion of the addition, the mixture was quenched by the addition of 3M HCl and extracted with EtOAc. The organic extract was washed successively with $H_2O$, aqueous $NaHCO_3$, $H_2O$ and saturated aqueous NaCl. The solvent was removed *in vacuo* to afford the title compound (11.58 g, 100%), which was used without further purification.

g) Methyl (RS)-5-benzyloxy-3-(4-methoxyphenyl)-1-(3,4-methylenedioxyphenyl)indene-2-carboxylate.

To a solution of methyl (3RS)-5-benzyloxy-3-hydroxy-3-(4-methoxyphenyl)-1-(3,4-methylenedioxyphenyl)indene-2-carboxylate (crude material prepared above) in $CH_2Cl_2$ (75 ml) under an argon atmosphere at 0°C was added triethylsilane (3.9 ml, 23.6 mmol), followed by boron trifluoride etherate (14.7 ml, 120 mmol). The reaction mixture was stirred for 10 min at 0°C, at which time the mixture was partitioned between 3M HCl and EtOAc. The organic extract was washed successively with $H_2O$, aqueous $NaHCO_3$, $H_2O$ and saturated aqueous NaCl and dried. The solvent was removed *in vacuo,* and the residue was purified by flash chromatography, eluting with a solvent gradient of 25 - 45% $Et_2O$/ hexanes. The title compound (8.41 g, 83% for two steps) was isolated as a mixture of Δ1 and Δ2 double bond isomers.

h) Methyl (1RS, 2RS, 3SR)-5-hydroxy-3-(4-methoxyphenyl)-1-(3,4-methylenedioxyphenyl)indane-2-carboxylate.

To a degassed solution of methyl (RS)-5-benzyloxy-3-(4-methoxyphenyl)-2-(3,4-methylenedioxyphenyl)indene-2-carboxylate (6.60 g, 13.0 mmol) in EtOAc (25 ml) and EtOH (175 ml) was added 5% palladium on activated carbon (0.6 g). The resulting suspension was shaken on a Parr hydrogenator at 60 psi $H_2$ for 20 h, at which time NMR analysis of the reaction mixture indicated that the reaction was incomplete. The catalyst was removed by filtration through a pad of Celite, and fresh 5% palladium on activated carbon (0.6 g) was added. The mixture was shaken on a Parr hydrogenator at 60 psi $H_2$ for an additional 48 h. The catalyst was removed by filtration through a pad of Celite, and the filtrate was concentrated under reduced pressure. The residue was crystallized from EtOAc/hexanes to afford the title compound (4.83 g, 89%); m.p. 187 - 188°C.

i) (1RS, 2SR, 3SR)-5-Hydroxy-3-(4-methoxyphenyl)-1-(3,4-methylenedioxyphenyl)indane-2-carboxylic acid, sodium salt.

To a solution of methyl (1RS, 2RS, 3SR)-5-hydroxy-3-(4-methoxyphenyl)-1-(3,4-methylenedioxyphenyl)indane-2-carboxylate (150 mg, 0.36 mmol) in EtOH (4 ml) was added 10% NaOH (4 ml), and the resulting mixture was allowed to stir under an argon atmosphere overnight. Water (5 ml) was added, and the mixture was concentrated under reduced pressure. The concentrate was extracted with $Et_2O$, and the aqueous phase was acidified and extracted with EtOAc. The EtOAc extract was washed successively with $H_2O$ and saturated aqueous NaCl and dried. The solvent was removed *in vacuo*. The sodium salt was prepared, and a portion of this (100 mg) was purified by reverse-phase chromatography to afford the title compound (73 mg, 48%). Trituration of this material with EtOAc provided a white crystalline solid; m.p. 198°C (dec). $^1$H NMR (MeOH-$d_4$): δ 7.20 (dd, 2H, *J* = 6.8 Hz, 2.0 Hz); 6.85 (dd, 2H, *J* = 6.8 Hz, 2.0 Hz); 6.80 - 6.64 (m, 5H); 6.25 (s, 1H); 5.88 - 5.87 (m, 2H); 4.47 (d, 1H, *J* = 10 Hz); 4.43 (d, 1H, *J* = 10 Hz); 3.76 (s, 3H); 3.03 (t, 1H, *J* = 10 Hz). MS (m/e): 427 [$(M+H)^+$].

EXAMPLE 12

(1RS,2SR,3RS)-3-(2-Carboxymethoxy-4-methoxyphenyl)-1-(3,4-methylenedioxyphenyl)-5-(prop-1-yloxy)-indane-2-carboxylic acid

a) 3-(Prop-1-yloxy)acetophenone.

To a slurry of NaH(13.84 g, 0.58 mol) in dry DMF (50 ml) at 0°C, was added a solution of 3-hydroxyacetophenone (50 g, 0.37 mol). After stirring for 30 min. 1-iodopropane (70 ml, 0.72 mol) was added and the mixture stirred overnight at room temperature. The mixture was diluted with dry DMF (50 ml) and further NaH (2.77 g, 0.12 mol) added

followed by 1-iodopropane (23 ml, 0.24 mol). After 1 h. TLC indicated that the reaction was complete and the product was cautiously quenched with 6M HCl and extracted with EtOAc. The EtOAc extract was washed successively with; $H_2O$, 10% aqueous NaOH and then brine. After drying ($MgSO_4$), filtration and evaporation gave the title compound (65 g, 98%) as a yellow oil which was used without further purification.

b) Methyl 3-(prop-1-yloxy)benzoylacetate.

To a suspension of NaH (12 g, 0.5 mol) in dry dimethyl carbonate (50 ml) was added slowly a solution of 3-(Prop-1-yloxy)acetophenone (65 g, 0.37 mol) in dry dimethyl carbonate (100 ml). During the addition the exothermicity of the reaction caused refluxing. Following the addition the mixture was stirred mechanically overnight and was then quenched cautiously with 3M HCl and extracted with EtOAc. The EtOAc extract was washed successively with; $H_2O$, 5% aqueous $NaHCO_3$, $H_2O$ and brine. After drying ($MgSO_4$), filtration and evaporation gave a yellow oil (82 g, quantitative) which was used without further purification.

c) Methyl (1RS,2SR)-1-(3,4-methylenedioxyphenyl)-5-(prop-1-yloxy)-3-oxo-indane-2-carboxylate.

To a solution of methyl 3-(prop-1-yloxy)benzoylacetate (10 g, 4.2 mmol) in benzene (50 ml) was added 3,4-methylene dioxybenzaldehyde (6.36 g, 4.2 mmol) followed by piperidine (0.42 ml, 0.42 mmol) and glacial acetic acid (8 drops approx.). The mixture was refluxed for 2 hr. and the volatiles removed *in vacuo* to give methyl (Z)-3-(3,4-methylenedioxyphenyl)-2-[3-(prop-1-yloxy)benzoyl]propenoate as a yellow oil. This residue was dissolved in trifluoroacetic acid (50 ml) and the mixture stirred at room temperature for 20 min. The trifluoroacetic acid was removed *in vacuo* to give the title compound as a dark oily residue (16 g) which was used in the next step without purification. $^1H$ NMR ($CDCl_3$): δ *inter alia* 7.85 (1H, s); 7.56-7.30 (3H, m); 7.08-7.15 (1H, m); 6.95 (1H, dd, J=8, 2Hz); 6.78.

d) Methyl 3-(3,4-methylenedioxyphenyl)-6-(prop-1-yloxy)-1-oxo-indene-2-carboxylate.

Methyl (1RS, 2SR)-1-(3,4-methylenedioxyphenyl)-5-(prop-1-yloxy)-3-oxo-indane-2-carboxylate (16 g, crude from previous experiment) was dissolved in dioxan (150 ml) and DDQ (22 g, 0.097 mol) added. The mixture was refluxed for 2 h. then cooled, filtered and the solvent removed *in vacuo.* The product was purified by flash column chromatography on silica gel (eluant: EtOAc/hexane, 20:80) to give the title compound as an orange solid (5.2 g, 31% over two steps); m.p. 125-126°C.

e) Methyl (1RS)-1-(2-benzvloxy-4-methoxyphenyl)-1-hydroxy-3-(3,4-methylenedioxyphenyl)-6-(prop-1 yloxy)indene-2-carboxylate.

To dry magnesium turnings (0.15 g, 6.25 mg. atoms) under an argon atmosphere was added portionwise, a solution of 2-benzyloxy-4-methoxybromobenzene (for preparation see below)(1.80 g, 6.15 mmol) in 5% THF/ether (7 ml). The resulting 2-benzyloxy-4-methoxyphenyl magnesium bromide was added to a solution of methyl-3-(3,4-methylenedioxyphenyl)-6-(prop-1-yloxy)-1-oxo-indene-2-carboxylate (1.5 g, 4.1mmol) in $Et_2O$ (65 ml) under an argon atmosphere at 0°C. The resulting mixture was allowed to warm to room temperature and was stirred for 10 min. The mixture was partitioned between 3M HCl (30 ml) and EtOAc (75 ml). The organic extract was washed successively with; $H_2O$, aqueous $NaHCO_3$, $H_2O$ and saturated aqueous NaCl and dried ($Na_2SO_4$). The solvent was removed under reduced pressure, and the residue purified by flash chromatography on silica gel (eluant: EtOAc/hexane, 30:70) to afford the title compound as a pale-yellow oil (1.4 g, 59%).

f) Methyl (RS)-3-(2-benzyloxy-4-methoxyphenyl)-1-(3,4-methylenedioxyphenyl)-5-(prop-1-yloxy)indene-2-carboxylate.

To a solution of (1.35 g, 2.33 mmol) in $CH_2Cl_2$ (20 ml) at 0°C under an argon atmosphere was added triethylsilane (0.47 ml, 2.94 mmol), followed by boron trifluoride etherate (1.4 ml, 11.4 mmol). The resulting solution was stirred at 0°C for 10 min, and was then partitioned between 1M HCl and EtOAc. The organic extract was washed successively with; $H_2O$, 5% aqueous $NaHCO_3$, $H_2O$ and brine. After drying ($Na_2SO_4$) the solvent was removed in vacuo, and the product purified by column chromatography on silica gel (eluant: EtOAc/hexane, 25:75). The title compound (as a single undefined double bond isomer) was obtained as yellow oil (0.65 g, 50%).

g) Methyl (1RS,2RS,3RS)-3-(2-hydroxy-4-methoxyphenyl)-1-(3,4-methylenedioxyphenyl)-5-(prop-1-yloxy)indane-2-carboxylate.

Methyl (RS)-3-(2-benzyloxy-4-methoxyphenyl) -1-(3,4-methylenedioxyphenyl)-5-(prop-1-yloxy)indene-2-carboxylate (0.64 g, 1.13 mmol) was dissolved in a small volume of EtOAc and EtOH (25 ml) added followed by 10% palladium on activated carbon (0.2 g). The resulting solution was stirred under an atmosphere of hydrogen for 10 days and filtered. The filtrate was concentrated under reduced pressure and the product purified by column chromatography on silica gel (eluant; EtOAc/hexane, 30:70) to give the title compound as a colorless solid (0.21 g, 39%); m.p. 155-156°C.

h) Methyl (1RS,2RS,3RS)-3-(2-carboethoxymethoxy-4methoxyphenyl)-1-(3,4-methylenedioxyphenyl)-5-(prop-1-yloxy)indane-2-carboxylate.

A solution of methyl (1RS,2RS,3RS)-3-(2-hydroxy-4-methoxyphenyl)-1-(3,4-methylenedioxyphenyl)-5-(prop-1-yloxy)indane-2-carboxylate (0.05 g, 0.11 mmol) in dry DMF (1 ml) was added to NaH (4 mg, 0.17 mmol) in a small volume of dry DMF. The mixture was stirred at room temperature for 10 min. and ethyl bromoacetate was added (0.016 ml, 0.14 mmol). After 20 min., the reaction was quenched with 3M HCl and extracted with EtOAc. The EtOAc extract was washed with water then brine, dried ($MgSO_4$), fitered and evaporated. The product was purified by column chromatography on silica gel (eluant: EtOAc/hexane, 30:70) to give the title compound as pale yellow oil (0.05g, 85%).

i) (1RS,2SR,3RS)-3-(2-Carboxymethoxy-4-methoxyphenyl)-1-(3,4-methylenedioxyphenyl)-5-(prop-1-yloxy)indane-2-carboxylic acid.

To a solution of methyl (1RS,2RS,3RS)-3-(2-carboethoxymethoxy-4-methoxyphenyl)-1-(3,4-methylenedioxyphenyl)-5-(prop-1-yloxy)indane-2-carboxylate (0.05 g, 0.089 mmol) in EtOH (1 ml) (warming necessary) was added 6M NaOH (0.089 ml, 0.53 mmol). After stirring overnight the product was partitioned between EtOAc and 3M HCl. The organic extract was washed with $H_2O$ and then brine, dried ($MgSO_4$), filtered and evaporated to give a colorless oil. The product was crystallized from $Et_2O$/hexane to give the title compound as an off-white solid (0.03 g, 65%); m.p. 195-198°C.
$^1H$ NMR [$(CD_3)_2CO$]: δ 7.17 (1H, d, J=9.1Hz); 6.8-6.71 (5H, m); 6.55-6.47 (3H, m); 5.94 (2H, s); 4.97 (1H, br. d); 4.73 (1H, d, J=16.5Hz); 4.63 (1H, d, J=16.5Hz); 4.52 (1H, d, J=7Hz); 3.80-3.76 (2H, m); 3.76 (3H, s); 3.48-3.35 (1H, br. m); 1.65 (2H, sextet, J=7.4Hz); 0.92 (3H, t, J=7.4Hz).MS: 538 [$(M+NH_4)^+$].
Anal. Calc. for $C_{29}H_{28}O_9$: C, 66.92; H, 5.42.
Found C, 67.37; H, 5.32.

EXAMPLE 12a

Preparation of 2-Benzyloxy-1-bromo-4-methoxybenzene.

a) 1-Bromo-2-hydroxy-4-methoxybenzene.

3-Bromo-2-hydroxy-6-methoxybenzoic acid [T. de Paulis *et al.,* J. Med. Chem., (1985), 28, 1263-1269](5 g, 0.02 mol) was heated in quinoline (200 ml) at 160°C for 1 h. On cooling, the product was partitioned between $Et_2O$ and 3M HCl. The organic extract was washed with water and brine then dried ($MgSO_4$), filtered and evaporated to give the title compound as a light-brown oil (4 g, 97%). This material was used without further purification.
$^1H$ NMR ($CDCl_3$): δ 7.32 (1H, d, J=9Hz); 6.60 (1H, d, J=1.5Hz), 6.43 (1H, dd, J=9,1.5Hz).

b) 2-Benzyloxy-1-bromo-4-methoxybenzene.

To a suspension of NaH (1.01 g, 0.042 mol) in dry DMF (ml) at 0°C was added solution of 1-bromo-2-hydroxy-4-methoxybenzene (7 g, 0.035 mol). After stirring at room temperature for 30 min. the solution was cooled to 0°C and benzyl bromide (6.24 ml, 0.052 mmol) added. The mixture was warmed to room temperature over 20 min. and then quenched cautiously by the addition of 3M HCl and extracted with EtOAc. The EtOAc extract was washed successively with; $H_2O$, 5% aqueous $NaHCO_3$, $H_2O$ and finally brine. After drying ($MgSO_4$) filtration and evaporation gave a dark colored oil. The product was purified by flash column chromatography (eluant: EtOAc/hexane, 20:80) to give the title compound as a colorless oil (7.5 g, 73%).
$^1H$ NMR ($CDCl_3$): δ 7.50-7.25 (6H, m); 6.51 (1H, d, J=1.5Hz); 6.39 (1H, d, J=9Hz); 5.09 (2H, s); 3.72 (3H, s).

EXAMPLE 13

(1RS, 2SR, 3RS)-3-F2-(3-Hydroxyprop-1-yloxy)-4-methoxyphenyl]-1-(3,4-methylenedioxyphenyl)-5-(prop-1-yloxy) indane-2-carboxylic acid, dicyclohexylamine salt

Methyl (1RS, 2RS, 3RS)-3-(2-hydroxy-4-methoxyphenyl)-1-(3,4-methylenedioxyphenyl)-5-(prop-1-yloxy)indane-2-carboxylate (0.14g, 0.29mmol) in dry DMF (1 ml) was added to NaH (9mg, 0.38mmol) in a small volume of dry DMF. The mixture was stirred at ambient temperature for 20 min. then 3-bromopropan-1-ol (37μl, 0.41mmol) was added. After stirring for 1h. the product was partitioned between 3M aqueous HCl and ethyl acetate. The organic layer was washed with water then brine, then dried ($MgSO_4$ anhyd.) filtered and evaporated to give an oil. The product was purified by column chromatography to provide methyl (1RS, 2SR, 3RS)-3-[2-(3-hydroxyprop-1-yloxy)-4-methoxyphenyl]-1-(3,4-methylenedioxyphenyl)-5-(prop-1-yloxy)indane-2-carboxylate (0.1g, 65%)($^1$H-NMR indicated some epimerization had occurred at C-2). This material was used without further purification. Methyl (1RS, 2SR, 3RS)-3-[2-(3-Hydroxyprop-1-yloxy)-4-methoxyphenyl]-1-(3,4-methylenedioxyphenyl)-5-(prop-1-yloxy)indane-2-carboxylate (0.04g, 0.075mmol) was dissolved in methanol (2ml) and aqueous potassium hydroxide added (2M, 0.22ml, 0.44mmol). The mixture was stirred under reflux overnight then cooled, diluted with water, acidified with 3M aqueous hydrochloric acid and extracted with ethyl acetate. The organic extract was washed with water and brine, dried ($MgSO_4$ anhydrous), filtered and evaporated to give an oil. The product was purified by chromatography on silica-gel (eluant: ethyl acetate/hexane/3% acetic acid) to give 12mg of free acid which was converted to its dicyclohexylamine salt. m.p. 110-112°C.

EXAMPLE 14

(1RS, 2SR, 3RS)-3-[2-(1-Carboxyeth-2-yloxy)-4-methoxyphenyl]-1-(3,4-methylenedioxyphenyl)-5-(prop-1-yloxy) indane-2-carboxylic acid, bis-dicyclohexylamine salt

(1RS, 2SR, 3RS)-3-[2-(3-Hydroxyprop-1-yloxy)-4-methoxyphenyl]-1-(3,4-methylenedioxyphenyl)-5-(prop-1-yloxy)indane-2-carboxylic acid (0.07g, 0.13mmol) was dissolved in dry dichloromethane (0.5ml) and Dess-Martin periodinane (0.07g, 0.17mmol) added in dry dichloromethane (1m1). After 2h. the product was partitioned between ether and saturated aqueous sodium carbonate solution containing sodium thiosulfate. The ether extract was washed with water then brine, dried ($MgSO_4$ anhydrous), filtered and evaporated to give an oil which was used without purification. The crude product was dissolved in t-butanol (5ml) and to this was added a solution of sodium chlorite (18mg, 0.2mmol) and sulfamic acid (21mg, 0.22mmol) in water (1.5ml). After 1h. stirring at ambient temperature the product was extracted into ethyl acetate. The organic layer was washed with water then brine then dried ($MgSO_4$ anhyd.) filtered and evaporated to give an oil. The product was purified by column chromatography on silica-gel (eluant: ethyl acetate/hexane/3% acetic acid) to give 12mg of free acid which was converted to its bis-dicyclohexylamine salt.
m.p. 160 - 162°C.
MS(exact mass) $M^+$: 534.1879 (free di-acid)
($\Delta$ = +1.1 mDa for $C_{30}H_{30}O_9$)

By the methods given above, the following compounds were made:

EXAMPLE 15

(1RS)-1-(4-Methoxyphenyl)-3-phenylindene-2-carboxylic acid

m.p. 191 - 193°C.
Anal. Calcd. for $C_{23}H_{18}O_3$: C, 80.68; H, 5.30.
Found: C, 80.54; H, 5.33.

EXAMPLE 16

(trans, trans)-1,3-Diphenylindane-2-carboxylic acid

m.p. 164 - 165°C.
MS (m/e) : 332 [$(M+NH_4)^+$].

EXAMPLE 17

(1RS, 2RS, 3SR)-1-(4-Hydroxyphenyl)-3-phenylindane-2-carboxylic acid

MS (m/e) : 331 $[(M+H)^+]$.

EXAMPLE 18

(1RS, 2RS, 3SR)-1-(4-Carboxyphenyl)-3-phenylindane-2-carboxylic acid

MS (m/e) : 359 $[(M+H)^+]$.

EXAMPLE 19

(1RS, 2RS, 3SR)-1-(3-Methoxyphenyl)-3-phenylindane-2-carboxylic acid

MS (m/e): 362 $[(M+NH_4)^+]$.

EXAMPLE 20

(1RS, 2RS, 3SR)-1-(4-Ethylphenyl)-3-phenylindane-2-carboxylic acid

m.p. 163 - 164°C.
MS (m/e): 360 $[(M+NH_4)^+]$.
Anal. Calcd. for $C_{24}H_{22}O_2$: C, 84.18; H, 6.48.
Found: C, 84.24; H, 6.73.

EXAMPLE 21

(1RS, 3RS)-1,3-Diphenylindane-2-carboxylic acid

m.p. 210-211°C.

EXAMPLE 22

(1RS, 2RS, 3SR)-1-(4-But-4-yloxyphenyl)-3-(4-methoxyphenyl)indane-2-carboxylic acid

$^1$H NMR ($CDCl_3$): δ 7.26 - 7.17 (m, 6H); 6.93 - 6.87 (m, 6H); 4.62 (d, 2H, *J* = 10.1 Hz); 3.96 (t, 2H, *J* = 6.5 Hz); 3.81 (s, 3H); 3.29 (t, 1H, *J* = 10.1 Hz); 1.80 - 1.73 (m, 2H); 1.54 - 1.45 (m, 2H); 0.98 (t, 3H, *J* = 7.3 Hz).

EXAMPLE 23

(1RS, 2RS, 3SR)-1-(4-Acetamidophenyl)-3-(4-methoxyphenyl)indane-2-carboxylic acid

m.p. 231 - 232°C.
MS (m/e, rel.int.): 803 $[(2M+1)^+$, 100].
Anal. Calcd. for $C_{25}H_{23}NO_4 \cdot 1/2\ H_2O$: C, 73.12; H, 5.85; N, 3.41. Found: C, 72.92; H, 5.61; N, 3.24.

EXAMPLE 24

(1RS, 2RS, 3SR)-1-(4-Aminophenyl)-3-(4-methoxyphenyl)indane-2-carboxylic acid, dicyclohexylamine salt

m.p. 187 - 190°C.
MS (m/e, rel.int.): 1076.2 $[(2M+1)^+$, 25].

EXAMPLE 25

(1RS, 2SR, 3SR)-1-(4-Hydroxyphenyl)-3-(3,4-methylenedioxyphenyl)indane-2-carboxylic acid

m.p. 94 - 96°C.
MS (m/e): 392.4 [$(M+NH_4)^+$].

EXAMPLE 26

(1RS, 2RS, 3SR)-1-(3,4-Dimethoxyphenyl)-3-(4-methoxyphenyl)indane-2-carboxylic acid

m.p. 126 - 128°C.
MS (m/e, rel.int.): 807 [$(2M+1)^+$, 35]; 403 [(M-H)-, 100] .
Anal. Calcd. for $C_{25}H_{24}O_5$: C, 74.24; H, 5.98. Found: C, 74.10; H, 5.99.

EXAMPLE 27

(1RS, 2RS, 3SR)-1-(3,4-Methylenedioxyphenyl)-3-(4-methylthiophenyl)indane-2-carboxylic acid

MS (exact mass): $(M\cdot)^+$ = 404.1074 (Δ = +0.8 mDa for $C_{24}H_{20}O_4S$).

EXAMPLE 28

(1RS, 2RS, 3SR)-5-Methoxy-3-(4-methoxyphenyl)-1-(3,4-methylenedioxyphenyl)indane-2-carboxylic acid

m.p. 129-131°C.
MS (m/e) : 441.2 [$(M+Na)^+$].

EXAMPLE 29

(1RS, 2SR, 3SR)-1,3-Bis(3,4-methylenedioxyphenyl)-5-hydroxyindane-2-carboxylic acid

MS (m/e): 436.2 [$(M+NH_4)^+$].

EXAMPLE 30

(1RS, 2SR, 3SR)-3-(2-Carboxymethoxy-4-methoxyphenyl)-1-(2-methoxy-4,5-methylenedioxyphenyl)-5-(prop-1-yloxy)indane-2-carboxylic acid

Methyl (1RS, 2RS, 3SR)-5-hydroxy-3-(2-methoxymethoxy-4-methoxyphenyl)-1-(2-methoxy-4,5-methylenedioxyphenyl)indane-2-carboxylic acid was prepared in 23% overall yield from methyl 2-(3-benzyloxy)benzoylacetate according to the method of example 11. The 5-hydroxyl moiety was then propylated according to the method given in example 12 and this crude material treated according to the method of example 70 to remove the methoxymethyl group in 55% yield. The title compound was then obtained following the procedure given for example 12 in 42% yield.
m.p. 188 - 190°C .
Anal. Calc. for $C_{30}H_{30}O_{10}$: C, 65.45; H, 5.49. Found: C, 65.38; H, 5.49.

EXAMPLE 31

(1RS, 2SR, 3RS)-3-(2-Methoxymethoxyl-4-methoxyphenyl)-1-(3,4-methylenedioxyphenyl)-5-(prop-1-yloxy)indane-2-carboxylic acid

m.p. 161 - 163°C.

EXAMPLE 32

(1RS, 2SR, 3RS)-3-(2-Hydroxy-4-methoxyphenyl)-1-(3,4-methylenedioxyphenyl)-5-(prop-1-yloxy)indane-2-carboxylic acid

(exact mass) $M^+$: 462.1678 ($\Delta$ = -0.4 mDa for $C_{27}H_{26}O_7$)

EXAMPLE 33

(1RS, 2SR, 3SR)-3-(2-Carboxymethoxy-4-methoxyphenyl)-1-[(2-prop-1-yloxy)-4,5-methylenedioxyphenyl]-5-(prop-1-yloxy)indane-2-carboxylic acid

Anal. Calc. for $C_{32}H_{34}O_{10}\cdot 0.5\ H_2O$: C, 65.41; H, 6.00. Found: C, 65.27; H, 5.99.
m.p. 196 - 197°C.

EXAMPLE 34

(1RS, 2SR, 3RS)-1-(2-Carboxymethoxy-4,5-methylenedioxyphenyl)-3-(4-methoxyphenyl)-5-(prop-1-yloxy)indane-2-carboxylic acid

MS (DCI $NH_3$) m/e: 538.2 $(M+NH_3)^+$, 520.2 $(M+H)^+$
(exact mass) $M^+$: 520.1733 ($\Delta$ = 0.0 mDa for $C_{29}H_{28}O_9$)

EXAMPLE 35

(1RS, 2SR, 3RS)-1-(3,4-Methylenedioxyphenyl)-3-[(2-prop-1-yloxy)phenyl]-5-(prop-1-yloxy)indane-2-carboxylic acid

m.p. 179 - 180°C.
MS (DCI $CH_4$) m/e: 503.2 $(M+C_2H_5)^+$, 474.1 $(M+H)^+$
(exact mass) $M^+$: 474.2034 ($\Delta$ = +0.8 mDa for $C_{29}H_{30}O_6$)

EXAMPLE 36

(1RS, 2SR, 3RS)-3-(2-Hydroxyphenyl)-1-(3,4-methylenedioxyphenyl)-5-(prop-1-yloxy)indane-2-carboxylic acid

m.p. 97 - 98°C.
MS (exact mass) $M^+$: 432.1568 ($\Delta$ = +0.5 mDa for $C_{26}H_{24}O_6$)

EXAMPLE 37

(1RS, 2SR, 3RS)-3-(2-Carboxymethoxyphenyl)-1-(3,4-methylenedioxyphenyl)-5-(prop-1-yloxy)indane-2-carboxylic acid

m.p. 169-170°C.
Anal. Calc. for $C_{28}H_{26}O_8\cdot 0.25\ H_2O$: C, 67.94; H, 5.40. Found: C, 67.75; H, 5.37.

EXAMPLE 38

(1RS, 2SR, 3RS)-3-(2-Benzyloxy-4-methoxyphenyl)-1-(3,4-methylenedioxyphenyl)-5-(prop-1-yloxy)indane-2-carboxylic acid

MS (exact mass) $M^+$: 552.2149 ($\Delta$ = -0.1 mDa for $C_{34}H_{32}O_7$)

EXAMPLE 39

(1RS, 2SR, 3RS)-3-[2-(2-Hydroxyeth-1-yloxy)-4-methoxyphenyl]-1-(3,4-methylenedioxyphenyl)-5-(prop-1-yloxy) indane-2-carboxylic acid, dicyclohexylamine salt

m.p. 182-184°C.

Anal. Calc. for $C_{41}H_{53}NO_8$: C, 71.59; H, 7.77; N, 2.04. Found: C, 71.67; H, 7.66; N, 2.42.

EXAMPLE 40

(1RS,2SR,3RS)-3-(2-Ethoxy-4-methoxyphenyl)-1-(3,4-methylenedioxyphenyl)-5-(prop-1-yloxy)indane-2-carboxylic acid

Anal. Calc. for $C_{29}H_{30}O_7$: C, 71.01; H, 6.16; Found: C, 70.71; H, 6.01.

EXAMPLE 41

(1RS, 2SR, 3RS)-3-[4-Methoxy-2-(prop-1-yloxy)]-1-(3,4-Methylenedioxyphenyl)-5-(prop-1-yloxy)indane-2-carboxylic acid

Anal. Calc. for $C_{30}H_{32}O_7$: C, 71.41; H, 6.39; Found: C, 71.43; H, 6.31.

EXAMPLE 42

(1RS, 2SR, 3RS)-3-[4-Methoxy-2-(prop-2-yloxy)phenyl]-1-(3,4-Methylenedioxyphenyl)-5-(prop-1-yloxy)indane-2-carboxylic acid

m.p. 75-79°C.

EXAMPLE 43

(1RS, 2SR, 3RS)-3-[4-Methoxy-2-(2-methylprop-1-yloxy)phenyl]-1-(3,4-Methylenedioxyphenyl)-5-(prop-1-yloxy) indane-2-carboxylic acid

m.p. 85-89°C.

EXAMPLE 44

(1RS, 2SR, 3RS)-3-[4-Methoxy-2-(3-methvlbut-1-yloxy)phenyl]-1-(3,4-methylenedioxyphenyl)-5-(prop-1-yloxy) indane-2-carboxylic acid, dicylohexylamine salt

m.p. 150-155°C.

EXAMPLE 45

(1RS, 2SR, 3RS)-3-[4-Methoxy-2-(3-pyridylmethoxy)phenyl]-1-(3,4-methylenedioxyphenyl)-5-(prop-1-yloxy)indane-2-carboxylic acid,

Anal. Calc. for $C_{33}H_{31}NO_7 \cdot 0.5H_2O$: C, 71.02; H, 5.78; N, 2.51; Found: C, 71.02; H, 5.53; H, 2.30.

EXAMPLE 46

(1RS, 2SR, 3RS)-3-[4-Methoxy-2-(4-pyridylmethoxy)phenyl]-1-(3,4-methylenedioxyphenyl)-5-(prop-1-yloxy)indane-2-carboxylic acid,

Anal. Calc. for $C_{33}H_{31}NO_7 \cdot 0.5H_2O$: C, 71.02; H, 5.78; N, 2.51; Found: C, 70.89; H, 5.59; H, 2.37.

EXAMPLE 47

(1RS, 2SR, 3RS)-3-[4-Methoxy-2-(2-pyridylmethoxy)phenyl]-1-(3,4-methylenedioxyphenyl)-5-(prop-1-yloxy)

m.p. 153-155°C.

EXAMPLE 48

(1RS,2SR,3RS)-3-[2-(Hept-1-yloxy)-4-methoxyphenyl]-1-(3,4-methylenedioxyphenyl)-5-(prop-1-yloxy)indane-2-carboxylic acid

m.p. 70-73°C.

EXAMPLE 49

(1RS, 2SR, 3RS)-3-[4-Methoxy-2-(5-tetrazolylmethoxy)phenyl]-1-(3,4-methylenedioxyphenyl)-5-(prop-1-yloxy) indane-2-carboxylic acid,

m.p. 102-105°C.

EXAMPLE 50

(1RS,2SR,3RS)-3-(2-Cyanomethoxy-4-methoxyphenyl)-1-(3,4-methylenedioxyphenyl)-5-(prop-1-yloxy)indane-2-carboxylic acid

m.p. 199-201°C.

EXAMPLE 51

(1RS,2SR,3RS)-3-(2-Carboxamidomethoxy-4-methoxyphenyl)-1-(3,4-methylenedioxyphenyl)-5-(prop-1-yloxy) indane-2-carboxylic acid

Anal. Calc. for $C_{29}H_{29}NO_8 \cdot 0.5C_4H_8O$: C, 67.02; H, 5.99; N, 2.52; ound: C, 67.76; H, 5.96; H, 2.56.

EXAMPLE 52

(1RS, 2SR, 3 SR)-5-Acetamido-1,3-bis(3,4-methylenedioxyphenyl)indane-2-carboxylic acid.

MS m/e: 460 [$(M+H)^+$].

EXAMPLE 53

(1RS, 2SR, 3SR)-5-Amino-1,3-bis(3,4-methylenedioxyphenyl)-indane-2-carboxylate, dicyclohexylamine salt.

MS m/e: 418 [$(M+H)^+$].

EXAMPLE 54

(1RS,2SR,3RS)-3-[2-(3-Carboxyphenyl)-4-methoxyphenyl]-1-(3,4-methylenedioxyphenyl)-5-(prop-1-yloxy)indane-2-carboxylic acid

a) Ethyl 3-[tri-(but-1-yl)stannyl)benzoate

Ethyl 3-bromobenzoate (2.0 g, 8.7 mmol), hexabutyldistannane (5.51 ml, 10.9 mmol), tetrakis(triphenylphosphine)palladium(0) (0.08 g, 0.07 mmol) and palladium (II) acetate (0.19 g, 0.85 mmol) were mixed in dry toluene (25 ml) and refluxed for 72 h under argon. The solvent was removed in vacuo and the residue purified by column chromatography on silica gel (eluant:hexane).
The title compound was obtained as a colorless oil (1.1 g, 30%).

b) Methyl (1RS,2SR,3RS)-3-[2-(3-carbomethoxyphenyl)-4-methoxyphenyl]-1-(3,4-methylenedioxyphenyl)-5-(prop-1-yloxy)indane-2-carboxylate

Methyl (1RS,2SR,3RS)-3-(4-methoxy-2-trifluoromethanesulfonyloxyphenyl)-1-(3,4-methylenedioxyphenyl)-5-(prop-1-yloxy)indane-2-carboxylate (0.118 g, 0.19 mmol), lithium chloride (0.058 g, 1.37 mmol), tetrakis(triphenylphosphine)palladium(0) (0.018 g, 0.016 mmol) and ethyl 3-[tri-(butyl-1-yl)stannyl]benzoate (0.253 g, 0.58 mmol) were mixed in dry dimethylformamide (5 ml) and refluxed for 24 h. The product was filtered through celite and the celite washed with ethyl acetate. The combined filtrate was evaporated in vacuo and was shown to be a mixture of two components by TLC. Purification by column chromatography on silica-gel gave a less polar fraction: methyl (1RS,2SR,3SR)-3-[2-(but-1-yl)-4-methoxyphenyl)-1-(3,4-methylenedioxyphenyl)-5-(prop-1-yloxy)indane-2-carboxylate (0.038 g) which was obtained as a colorless oil. The title compound was the more polar component (0.08g) which while contaminated with tin residues ($^1$H-NMR) was used without further purification.

c) (1RS,2SR,3RS)-3-[2-(3-Carboxyphenyl)-4-methoxyphenyl]-1-(3,4-methylenedioxyphenyl)-5-(prop-1-yloxy)indane-2-carboxylic acid

Methyl (1RS,2SR,3RS)-3-[2-(3-Carbomethoxyphenyl)-4-methoxyphenyl]-1-(3,4-methylenedioxyphenyl)-5-(prop-1-yloxy)indane-2-carboxylate (0.08g, crude) was dissolved in propan-2-ol (1 ml) and aqueous sodium hydroxide (1M, 1 ml ,1 mmol) added. The mixture was refluxed for 12 hr. then cooled, diluted with water, acidified with 3M-aqueous hydrochloric acid and extracted with ethyl acetate (3x). The combined organic extract was purified by column chromatography on silical-gel (eluant: 30% EtOAc/hexane/5%AcOH) to give the title compound as a colorless solid (20 mg) m.p. 257-268°C.

EXAMPLE 55

(1RS,2SR,3SR)-3-[2-(But-1-yl)-4-methoxyphenyl)-1-(3,4-methylenedioxyphenyl)-5-(prop-1-yloxy)indane-2-carboxylic acid, dicyclohexylamine salt

Methyl (1RS,2SR,3SR)-3-[2-(but-1-yl)-4-methoxyphenyl)-1-(3,4-methylenedioxyphenyl}-5-(prop-1-yloxy)indane-2-carboxylate (0.038g, 0.074 mmol) was dissolved in propan-2-ol (1 ml) and aqueous sodium hydroxide (1M, 0.75 ml, 0.75 mmmol) added. The mixture was refluxed for 12 hr. then cooled, diluted with water, acidified with 3M-aqueous hydrochloric acid and extracted with ethyl acetate (3x). The combined organic extract was purified by column chromatography on silica-gel (eluant: 30% EtOAc/hexane then 30% EtOAc/hexane/5%AcOH). Conversion of the product to its dicyclohexylamine salt gave the title compound.
m.p. 179-182°C.
Anal. Calc. for $C_{41}H_{53}NO_8$: C, 71.59; H, 7.77; N, 2.04. Found: C, 71.67; H, 7.66; N, 2.42.

EXAMPLE 56

(1RS,2SR,3SR)-3-(4-Methoxy-2-phenylphenyl)-1-(3,4-methylenedioxyphenyl)-5-(prop-1-yloxy)indane-2-carboxylic acid

a) Methyl (1RS,2RS,3SR)-3-(4-methoxy-2-phenylphenyl)-1-(3,4-methylenedioxyphenyl)-5- (prop-1-yloxy) indane-2-carboxylate

To a slurry of anhydrous LiCl (46 mg, 1.1 mmol) and tetrakis(triphenylphosphine)palladium(0)(24 mg, 0.02 mmol) in dry dioxane (3 mL) was added a solution of Methyl (1RS,2RS,3RS)-3-(4-methoxy-2-trifluoromethanesulfonyloxyphenyl)-1-(3,4-methylenedioxyphenyl)-5-(prop-1-yloxy)indane-2-carboxylate (95 mg, 0.16 mmol) and tri(but-1-yl)stannylbenzene (319 mg, 0.87 mmol) in dioxane (1 mL). The mixture was refluxed under Argon for 17 h, cooled to room temperature, diluted with ethyl acetate (5 ml) and the resulting solution washed sequentially with brine and water. The organic layer was dried ($MgSO_4$ anhydrous), filtered through a short pad of silica gel and concentrated *in vacuo* to yield an oil. The product was purified by flash column chromatography (silica gel, gradient elution from hexanes to 10 % ethyl acetate/hexanes) to afford the title compound as a white solid. (92 mg, 86%).

b) (1RS,2SR,3SR)-3-(4-Methoxy-2-phenylphenyl)-1-(3,4-methylenedioxyphenyl)-5-(prop-1-yloxy)indane-2-carboxylic acid

To a solution of Methyl (1RS,2RS,3SR)-3-(4-methoxy-2-phenylphenyl)-1-(3,4-methylenedioxyphenyl)-5-

(prop-1-yloxy)indane-2-carboxylate (80 mg, 0.12 mmol) in dioxane (2 mL) was added 1M aqueous NaOH (0.3 mL, 0.3 mmol). The resulting mixture was heated to reflux for 48 h, then concentrated under reduced pressure. The residue was partitioned between dilute aqueous HCl and ethyl acetate. The ethyl acetate extract was washed with water and dried ($MgSO_4$ anhydrous). The solvent was removed *in vacuo* and the residue purified by flash column chromatography (silica gel, 20% ethyl acetate/hexane containing 5% of acetic acid) to afford the title compound (36 mg, 46%).
m.p. 199 - 200°C.
$^1$H NMR ($CDCl_3$) δ 7.18-7.09 (m, 6H); 6.85 (dd, 1H, J = 8.6, 2.1 Hz); 6.71-6.65 (m, 6H),6.36 (b s, 1H), 5.85 (s, 2H), 4.59 (d, 1H, *J* = 10.2 Hz); 4.31 (d, 1H, J = 10.2 Hz); 3.75 (t, 2H, J = 7.3 Hz); 3.73 (s, 3H); 3.14 (dd, 1H, J = 10.2, 10.2 Hz); 1.68 (sextet, 2H, J = 7.3 Hz); 0.93 (t, 3H, J = 7.3 Hz).
MS m/e: 540 $(M+NH_4)^+$.
Anal. Calc. for $C_{33}H_{30}O_6$ 3/4 $H_2O$: C, 73.93; H, 5.90. Found: C, 74.12, H, 5.80.

EXAMPLE 57

(1RS, 2SR, 3SR)-3-[2-[(E)-2-Carboxyethen-1-yl]-4-methoxyphenyl]-1-(3,4-methylenedioxyphenyl)-5-(prop-1-yloxy) indane-2-carboxylic acid

a) Methyl (1RS, 2SR, 3SR)-3-[2-[(E)-2-carbomethoxyethen-1-yl]-4-methoxyphenyl]-1-(3,4-methylenedioxyphenyl)-5-(prop-1-yloxy)indane-2-carboxylate.

1,3-bis(diphenylphosphino)propane (0.066 mmol), tris(dibenzylideneacetone)dipalladium(0)(24 mg, 0.026) and bis(triphenylphosphine)palladium(II) choride (18 mg, 0.026 mmol), were dissolved in a 4:1 mixture of triethylamine/acetonitrile (5 mL) under argon. After 10 min at room temperature, a solution of methyl (1RS, 2SR, 3RS)-3-(4-methoxy-2-trifluoromethanesulfonyloxyphenyl)-1-(3,4-methylenedioxyphenyl)-5-(prop-1-yloxy)indane-2-carboxylate (160 mg, 0.26 mmol) and methyl acrylate (679 mg, 7.89 mmol) was added in the above solvent mixture (3 mL). The reaction mixture was heated to reflux under argon for 20 h, cooled to room temperature and a small aliquot analyzed by $^1$H NMR, which showed no reaction had taken place. Palladium(II) acetate (6 mg, 0.025 mmol) and methyl acrylate (679 mg, 7.89 mmol) in dry DMF (5 mL) were then added. The reaction mixture was heated to reflux overnight. On cooling the solution was filtered through a short column of silica gel and concentrated to yield an oil. The crude product was purified by flash column chromatography (silica gel, gradient elution: 10 % to 20% ethyl acetate/hexanes) to afford the title compound as a tan solid. (87 mg, 62%).
$^1$H NMR ($CDCl_3$) : δ 8.17 (d, 1H, J = 15.7 Hz); 7.44 (d, 1H, J = 8.7 Hz), 7.11-7.07 (m, 2H); 6.90-6.70 (m, 6H), 6.42 (d, 1H, J = 15.7 Hz); 5.94 (b s, 2H), 5.04 (d, 1H, J = 7.5 Hz); 4.75 (d, 1H, J = 7.6 Hz); 3.89 (t, 2H, J = 6.7 Hz); 3.85 (s, 3H); 3.85 (dd, 1H, J = 7.5, 7.4 Hz); 3.83 (s,3H); 2.96 (s,3H), 1.79 (sextet, 2H, J = 6.7 Hz); 1.03 (t, 3H, J = 6.7 Hz).

b) (1RS, 2SR, 3SR)-3-[2-[(E)-2-Carboxyethen-1-yl]-4-methoxyphenyl]-1-(3,4-methylenedioxyphenyl)-5-(prop-1-yloxy) indane-2-carboxylic acid.

To a solution of methyl (1RS, 2SR, 3SR)-3-[2-[(E)-2-carbomethoxyethen-1-yl]-4-methoxyphenyl]-1-(3,4-methylenedioxyphenyl)-5-(prop-1-yloxy)indane-2-carboxylate (80 mg, 0.15 mmol) in dioxane (2 ml) was added 1 N NaOH (0.5 ml, 0.5 mmol). The resulting mixture was heated to reflux for 3 h, then cooled and concentrated under reduced pressure. The residue was partitioned between dilute aqueous HCl and ethyl acetate. The ethyl acetate extract was washed with water and dried ($MgSO_4$ anhydrous). The solvent was removed *in vacuo* and the title compound was obtained as a white solid (73 mg, 96%).
$^1$H NMR ($CDCl_3$): δ 8.32 (d, 1H, J = 15.6 Hz); 7.24-6.55 (m, 9H); 6.29 (d, 1H, J = 15.6 Hz); 5.94 (b s, 2H), 5.18 (d, 1H, J = 9.9 Hz); 4.69 (d, 1H, J = 9.9 Hz); 3.85 (s, 3H); 3.84 (t, 2H, J = 6.9 Hz); 2.94 (dd, 1H, J = 9.9, 9.9 Hz); 1.79 (sextet, 2H, J = 6.9 Hz); 1.00 (t, 3H, J = 6.9 Hz).
MS m/e: 517 [$(M+H)^{+}$].
Anal. Calc. for $C_{30}H_{28}O_8$: C, 69.76; H, 5.46. Found: C, 69.73, H, 5.26.

EXAMPLE 58

(1RS, 2SR, 3SR)-3-[2-(2-Carboxyeth-1-yl)-4-methoxyphenyl]-1-(3,4-methylenedioxyphenyl)-5-(prop-1-yloxy)indane-2-carboxylic acid.

To a solution of (1RS, 2SR, 3 SR)-3-[2-[(E)-2-carboxyethen-1-yl]-4-methoxyphenyl]-1-(3,4-methylenedioxyphenyl)-5-(prop-1-yloxy)indane-2-carboxylic acid (43 mg, 0.08 mmol) in ethanol (5 mL) was added 10% palladium on activated carbon (40 mg). The resulting suspension was stirred overnight under an atmosphere of hydrogen then

filtered through a pad of celite. The filtrate was concentrated under reduced pressure to afford the title compound (35 mg, 82%) as a white solid.

$^1$H NMR ($CDCl_3$): δ 6.99 (d, 1H, J = 8.6 Hz); 6.78-6.66 (m, 7H); 6.23 (b s, 1H); 5.88-5.87 (m, 2 H); 4.88 (d, 1H, J = 9.7 Hz); 4.54 (d, 1H, J = 9.7 Hz); 3.72 (s, 3H); 3.70 (t, 2H, J = 7 Hz); 2.98-2.90 (m, 1H); 2.68-2.51 (m, 2H); 1.65 (sextet, 2H, J = 7.0 Hz); 0.89 (t, 3H, J = 7.0 Hz).

MS (exact mass) $M^+$: 518.1930 (Δ = +1.1 mDa for $C_{27}H_{26}O_7$)

By the methods given above in Examples 54 to 58, the following compounds were made.

EXAMPLE 59

(1RS,2SR,3RS)-3-(2-Carboxymethylthio-4-methoxyphenyl)-1-(3,4-methylenedioxyphenyl)-5-(prop-1-yloxy)indane-2-carboxylic acid

m.p. 242-246°C (dec).

EXAMPLE 60

(1RS, 2SR, 3SR)-3-[4-Methoxy-2-(prop-2-en-1-yl)phenyl]-1-(3,4-methylenedioxyphenyl)-5-(prop-1-yloxy)indane-2-carboxylic acid

m. p. 126-127°C.

(exact mass) $M^+$: 486.2021 (△ = +2.1 mDa for $C_{30}H_{30}O_6$)

EXAMPLE 61

(1RS, 2SR, 3SR)-3-[4-Methoxy-2-(prop-1-yl)phenyl]-1-(3,4-methylenedioxyphenyl)-5-(prop-1-yloxy)indane-2-carboxylic acid

m.p. 155-156°C.

Anal. Calc. for $C_{30}H_{32}O_6$: C, 73.75; H, 6.60. Found: C, 73.45, H, 6.43.

EXAMPLE 62

(1RS, 2SR, 3RS)-3-[2-Carboxy-4-methoxyphenyl]-1-(3,4-methylenedioxyphenyl)-5-(prop-1-yloxy)indane-2-carboxylic acid.

Anal. Calc. for $C_{28}H_{26}O_8$: C, 68.56; H, 5.34. Found: C, 68.61, H, 5.58.

EXAMPLE 63

(1RS, 2SR, 3 SR)-3-[2-(2-Hydroxyethyl)-4-methoxyphenyl]-1-(3,4-methylenedioxyphenyl)-5-(prop-1-yloxy)indane-2-carboxylic acid

(exact mass) $M^+$: 490.1994 (△ = +0.3 mDa for $C_{29}H_{30}O_7$)

EXAMPLE 64

(1RS, 2SR, 3 SR)-3-(2-Carboxymethyl-4-methoxyphenyl)-1-(3,4-methylenedioxyphenyl)-5-(prop-1-yloxy)indane-2-carboxylic acid

(exact mass) $M^+$: 504.1788 (Δ = -0.4 mDa for $C_{29}H_{28}O_8$)

EXAMPLE 65

(1RS, 2SR, 3SR)-3-[2-(3-Hydroxyprop-1-yl)-4-methoxyphenyl]-1-(3,4-methylenedioxyphenyl)-5-(prop-1-yloxy) indane-2-carboxylic acid

MS (exact mass) $M^+$: 504.2143 (Δ = +0.5 mDa for $C_{30}H_{32}O_7$)

EXAMPLE 66

(1RS, 2SR, 3 SR)-5-(4-Carboxyphenyl)-1,3-bis(3,4-methylenedioxyphenyl)1-indane-2-carboxylic acid.

m.p. 230-231°C.

EXAMPLE 67

(1RS, 2SR, 3SR)-5-(4-Benzyloxyphenyl)-1,3-bis(3,4-methylenedioxyphenyl)indane-2-carboxylic acid.

m.p. 105-106°C.

EXAMPLE 68

(1RS, 2SR, 3SR)-5-([4-Hydroxyphenyl)-1,3-bis(3,4-methylenedioxyphenyl)indane-2-carboxylic acid.

MS m/e: 512 $[(M+NH_4)^+]$.

EXAMPLE 69

(trans, trans-1,3,5-Tris(3,4-methylenedioxyphenyl)indane-2-carboxylic acid.

Anal. Calc. for $C_{31}H_{22}O_8.5/8H_2O$: C, 69.76; H, 4.39. Found: C, 69.81, H, 4.46.

EXAMPLE 70

(1RS,3RS)-3-(2-Hydroxy-4-methoxyphenyl)-1-(3,4-methylenedioxyphenyl)-5-(prop-1-yloxy)indane.

a) (1RS,3RS)-3-[(2-Methoxymethoxy)-4-methoxyphenyl)]-1-(3,4-methylenedioxyphenyl)-5-(prop-1-yloxy)indane.

A solution of (1RS, 2SR, 3RS)-3-[2-(methoxymethoxy)-4-methoxyphenyl]-1-(3,4-methylenedioxyphenyl)-5-(prop-1-yloxy)indane-2-carboxylic acid (0.2g, 0.39mmol) in dichloromethane (4ml) and pyridine (28µl, 1.6 mmol) was cooled to 0 °C under argon. To this solution was added thionyl chloride (60 µl, 0.8 mmol). The mixture was allowed to warm to ambient temperature over 20 min. and the volatiles removed in vacuo. The residue was redissolved in toluene and evaporated in vacuo (twice). The residue was dissolved in dichloromethane (4ml) and triethylamine (250µl) added. To this solution at room temperature under argon was added 2-mercaptopyridine-N-oxide (120 mg, 0.8mmol) dissolved in dichloromethane (1m1). After stirring for 20 min at room termperature t-butylthiol (450µl, 4mmol) was added and the mixture irradiated for 20 min (150 watt spotlight). The volatiles were removed in vacuo and the product partitioned between ethyl acetate and 3-M-aq. HCl. The organic extract was washed with water, sat. aq. $NaHCO_3$ solution and finally brine. After drying ($MgSO_4$ anhydrous), the product was filtered and evaporated. Purification by column chromatography gave the title compound (0.075 g, 41%).
$^1$H NMR ($CDCl_3$) : δ 7.13 (d, 1H, J = 8.5 Hz); 6.83 (d, 1H, J = 8.3 Hz), 6.79-6.69 (m, 5H), 6.54 (dd, 1H, J = 8.5, 2.5 Hz), 6.51 (br s, 1H), 5.92 (br, s, 2H) 5.18 (d, 1H, J = 6.7 Hz, ), 5.15 (d, 1H, J = 6.7 Hz), 4.66 (dd, J = 10.5, 7.6 Hz, 1H, J = 6.7 Hz), 4.22 (dd, 1H, J = 10.5, 7.4 Hz), 3.81 (m, 2H), 3.80 (s, 3H), 3.43 (s, 3H), 2.90-2.83 (m, 1H), 2.06-1.98 (m, 1H), 1.73 (sextet, 1H, J = 7.1 Hz), 0.92 (t, 3H, J = 7.1 Hz).

b) (1RS,3RS)-3-(2-Hydroxy-4-methoxyphenyl)-1-(3,4-methylenedioxyphenyl)-5-(prop-1-yloxy)indane

To a solution of (1RS,3RS)-3-[(2-methoxymethoxy)-4-methoxyphenyl)]-1-(3,4-methylenedioxyphenyl)-5-(prop-1-yloxy)indane (0.075 g, 0.16 mmol) in methanol (5 ml) was added 4-5 drops of 6M-HCl and the mixture refluxed for 1.5 h under argon. The solvent was removed in vacuo and the product partitioned between EtOAc and water. The organic extract was washed with water then sat. aq. $NaHCO_3$ solution and finally brine. After drying ($MgSO_4$ anhydrous) filtration and evaporation gave the title compound (0.064 g, 94%).
$^1$H NMR ($CDCl_3$): δ 7.11 (d, 1H, J = 8.4 Hz), 6.87 (d, 1H, J = 7.8 Hz), 6.77-6.74 (4 H, m), 6.61 (br s, 1H), 6.50 (dd, 1H, J = 8.4, 2.5 Hz), 6.42 (d, 1H, J = 2.5 Hz), 5.94 (d, 1H J = 1.2 Hz), 5.93 (d, 1H, J = 1.2 Hz), 4.74 (s, 1H), 4.43 (dd, 1H, J = 10.4, 7.6 Hz), 4.20 (dd, 1H, J = 10.7, 7.3 Hz), 3.82 (t, 2H, J = 6.7 Hz), 3.79 (s, 3H), 2.89-2.82 (m, 1H), 2.15-2.08 (m, 1H), 1.77-1.71 (sextet, 2H, J = 7.2 Hz), 0.99 (t, 3H, J = 2.5 Hz).
MS (exact mass) M+ Found: 418.1782 (Δ = -0.2 mDa for $C_{26}H_{26}O_5$).

EXAMPLE 71

(1RS,2RS)-3-(2-Carboxymethoxy-4-methoxyphenyl)-1-(3,4-methylenedioxyphenyl)-5-(prop-1-yloxy)indane

To a slurry of sodium hydride (5 mg, 0.21 mmol) in dimethylformamide (0.5 ml) was added (1RS,3RS)-3-(2-hydroxy-4-methoxyphenyl)-1-(3,4-methylenedioxyphenyl)-5-(prop-1-yloxy)indane(0.058 g, 0.14 mmol) at ice-bath temperature under argon. After stirring for 15 min, ethyl bromoacetate (50 µl, 0.2 mmol) was added and the solution stirred for 1 h at room temperature. The product was partitioned between ethyl acetate and 3M aq HCl. The organic extract was washed with water, sat. aq. $NaHCO_3$ solution and finally brine. After drying ($MgSO_4$ anhydrous) filtration and evaporation followed by chromatography gave (1RS,3RS)-3-(2-carboethoxymethoxy-4-methoxyphenyl)-1-(3,4-methylenedioxyphenyl)-5-(prop-1-yloxy)indane (0.041 g). The product was dissolved in hot ethanol (10 ml) and 1 M aq. NaOH added (1 ml). The mixture was refluxed for 1 h then cooled, acidified with 6M-aqueous HCl and extracted with ethyl acetate. After evaporation the residue was crystallized from ethyl acetate/hexane to give the title compound (0.035 g, 93%). m.p. 177-178°C.
$^1$H NMR ($CDCl_3$): δ 7.18 (d, 1H, J = 8.5 Hz), 6.87 (d, 1H, J = 8.4 Hz), 6.88-6.71 (4 H, m), 6.56 (dd, 1H, J = 8.4, 2.3 Hz), 6.53 (br. s, 1H), 6.41 (d, 1H, J = 2.3 Hz), 5.91 (br. s, 2H), 4.68-4.60 (m, 3H), 4.61 (dd, 1H, J = 10.7, 7.2 Hz), 3.83-3.80 (m, 2H), 3.81 (s, 3H), 2.86 (dt, 1H, J = 12.4, 7.2 Hz), 2.10-1.98 (m, 1H), 1.73 (sextet, 2H, J = 7.2 Hz), 0.98 (t, 3H, J = 7.4 Hz).
MS (exact mass) M+ = 476.1829 (Δ = $^+$0.6 mDa for $C_{28}H_{28}O_7$).

EXAMPLES 72-84

The following compounds were prepared by the procedures given above.

(1RS, 2SR, 3SR)-1-(4-Methoxyphenyl)-3-(3,4,5-trimethoxyphenyl)indane-2-carboxylic acid;

(1RS, 2SR, 3SR)-1-(4-Ethoxyphenyl)-3-(3-4-methylenedioxyphenyl)indane-2-carboxylic acid;

(1RS, 2SR, 3SR)-5-Carboxy-1,3-bis(3,4-methylenedioxyphenyl)indane-2-carboxylic acid;

(1RS, 2SR, 3SR)-3-(4-Methoxyphenyl)-1-(3,4-methylenedioxyphenyl)-5-(prop-2-enyloxy)indane-2-carboxylic acid;

(1RS, 2SR, 3RS)-3-(2,4-Dimethoxyphenyl)-5-hydroxy-1-(3,4-methylenedioxyphenyl)-indane-2-carboxylic acid;

(1RS, 2SR, 3SR)-3-[5-(2,3-Dihydro)benzfuranyl]-5-hydroxy-1- (3,4-methylenedioxyphenyl)indane-2-carboxylic acid;

(1RS, 2SR, 3RS)-5-Hydroxy-3-(3,4-methylenedioxyphenyl)-1-(2,4,6-trimethoxyphenyl)indane-2-carboxylic acid;

(1RS, 2SR, 3SR)-1-[5-(2,3-Dihydro)benzfuranyl]-1-(4-methoxyphenyl)indane-2-carboxylic acid;

(1RS, 2SR, 3RS)-1-[3,4-(1,2-Ethylenedioxy)phenyl]-3-(4-methoxyphenyl)indane-2-carboxylic acid;

(1RS, 2SR, 3SR)-5-Hydroxy-3-(3,4-methylenedioxyphenyl)-1-(4-methoxyphenyl)indane-2-carboxylic acid;

(1RS, 2SR, 3RS)-5-Hydroxy-3-(4-methoxyphenyl)-1-(2-methoxy-4,5-methylenedioxyphenyl)indane-2-carboxylic acid;

(1RS, 2SR, 3SR)-1-(3,4-Methylenedioxyphenyl)-3-(4-methoxyphenyl)-5-(propyl-1-yloxy)indane-2-carboxylic acid;

(1RS, 2SR, 3RS)-5-Methoxy-3-(4-methoxyphenyl)-1-(2-methoxy-4,5-methylenedioxyphenyl)indane-2-carboxylic acid.

EXAMPLE 85

Formulations for pharmaceutical use incorporating compounds of the present invention can be prepared in various forms and with numerous excipients. Examples of such formulations are given below.

Inhalant Formulation

A compound of formula I, (1 mg to 100 mg) is aerosolized from a metered dose inhaler to deliver the desired amount of drug per use.

| Tablets/Ingredients | | Per Tablet |
|---|---|---|
| 1. | Active ingredient (Cpd of Form. I) | 40 mg |
| 2. | Corn Starch | 20 mg |
| 3. | Alginic acid | 20 mg |
| 4. | Sodium alginate | 20 mg |
| 5. | Mg stearate | 1.3 mg |
| | | 2.3 mg |

Procedure for tablets:

Step 1 Blend ingredients No. 1, No. 2, No. 3 and No. 4 in a suitable mixer/blender.
Step 2 Add sufficient water portion-wise to the blend from Step 1 with careful mixing after each addition. Such additions of water and mixing until the mass is of a consistency to permit its conversion to wet granules.
Step 3 The wet mass is converted to granules by passing it through an oscillating granulator using a No. 8 mesh (2.38 mm) screen.
Step 4 The wet granules are then dried in an oven at 140°F (60°C) until dry.
Step 5 The dry granules are lubricated with ingredient No. 5.
Step 6 The lubricated granules are compressed on a suitable tablet press.

Parenteral Formulation

A pharmaceutical composition for parenteral administration is prepared by dissolving an appropriate amount of a compound of formula I in polyethylene glycol with heating. This solution is then diluted with water for injections Ph Eur. (to 100 ml). The solution is then sterilized by filtration through a 0.22 micron membrane filter and sealed in sterile containers.

**Claims**

**1.** A compound of Formula (I):

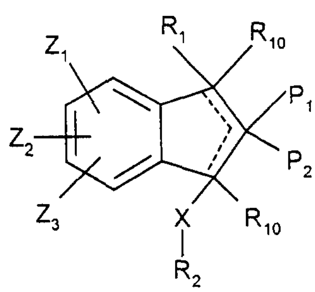

(I)

wherein $R_1$ is $X(CH_2)_nAr$, dihydrobenzofuranyl, benzodioxanyl, cyclohexyl, or $C_{1-4}$alkyl;
$R_2$ is a moiety of formula (a) or (b), $C_{1-4}$alkyl, indolyl or hydrogen;
$R_3$ and $R_5$ are independently hydrogen, OH, $C_{1-5}$alkoxy, halogen, $-OC_{1-4}$alkyl phenyl, $R_{11}CO_2R_7$, $C_{1-4}$alkyl, $N(R_6)_2$, $NH(CO)CH_3$, $-X(CH_2)_nR_8$, $-X-R_9-Y$, pyridyl, phenyl or $S(O)_qC_{1-5}$alkyl;
$R_4$ is hydrogen, OH, $C_{1-5}$alkoxy, halogen, $C_{1-4}$alkyl, $N(R_6)_2$, $NH(CO)CH_3$ or $S(O)_qC_{1-5}$alkyl;

$P_1$ and $P_2$ are independently hydrogen, $CO_2H$ or tetrazolyl;
$R_6$ is independently hydrogen or $C_{1-4}$alkyl;
$R_7$ is independently hydrogen, $C_{1-6}$alkyl or $(CH_2)_nAr$;
$R_8$ is $C_{1-8}$alkyl, $C_{2-8}$alkenyl or $C_{2-8}$alkynyl, all of which may be unsubstituted or substituted by one or more OH, $CH_2OH$, $N(R_6)_2$ or halogen; hydrogen, $CO_2H$, $PO_3H_2$, $P(O)(OH)R_7$ or tetrazole;
$R_9$ is $C_{1-10}$alkylene, $C_{2-10}$alkenylene or phenylene, all of which may be unsubstituted or substituted by one or more OH, $N(R_6)_2$, COOH, halogen or $XC_{1-5}$alkyl;
$R_{10}$ is hydrogen;
$R_{11}$ is $C_{1-8}$alkylene, $C_{2-8}$alkenylene or $C_{2-8}$alkynylene, all of which may be unsubstituted or substituted by one or more OH, $CH_2OH$, $N(R_6)_2$ or halogen;
X is $(CH_2)_n$ or oxygen;
Y is $CH_3$ or $-X(CH_2)_nAr$;
Ar is:

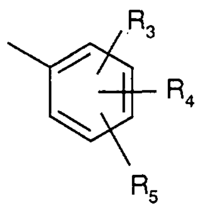
(a),

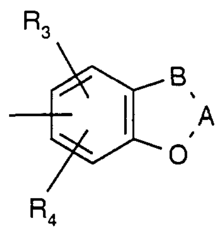
(b),

or pyridyl optionally substituted by one or more $R^3$ or $R^4$ groups;
A is C=O or $[C(R_6)_2]_m$;
B is $-CH_2-$ or -O-;
$Z_1$, $Z_2$ and $Z_3$ are independently $-X-R_9-Y$, benzyl, hydrogen, OH, $C_{1-5}$alkoxy, $-N(R_6)_2$, $S(O)_qC_{1-8}$alkyl, $NHCOR_6$, $X(CH_2)_nR_8$ or halogen, or $Z_1$ and $Z_2$ together may be -O-A-O- on contiguous carbons;
q is zero, one or two;
n is an integer from 0 to six;
m is 1, 2 or 3; and the dotted line indicates the optional presence of a double bond; or a pharmaceutically acceptable salt thereof;

provided that a) when the optional double bond is present there is only one $R_{10}$ and there is no $P_1$; and b) the compound of Formula I is not:

(1RS)-1,3-diphenylindene-2-carboxylic acid;
(cis,cis)-(1RS,3SR)-1,3-diphenylindane-2-carboxylic acid;
1,3-diphenylindene;
1,3-diphenylindane; or
1,3-bis(3,4-dimethoxyphenyl)-5,6-dimethoxyindane.

**2.** A compound according to claim 1 wherein:

$R_3$ is hydrogen, $-X(CH_2)_nR_8$ or $R_{11}CO_2R_7$;
$R_4$ and $R_5$ are independently hydrogen, OH, $C_{1-5}$ alkoxy, $SC_{1-5}$ alkyl, F, Br, $C_{1-3}$ alkyl or $NH_2$; and
$Z_1$ and $Z_3$ are hydrogen and $Z_2$ is hydrogen, OH, $C_{1-5}$ alkoxy, halogen, $X(CH_2)_nR_8$, $NH_2$, benzyl or NH(CO)$CH_3$, or $Z_1$ and $Z_2$ together may be -O-A-O-on contiguous carbons.

**3.** A compound according to claim 1 or claim 2 wherein:

$R_1$ is a moiety of formula (b) and $R_2$ is a moiety of formula (a) or (b);
A is $CH_2$;
B is -O-;

there is no optional double bond;

$R_1$ and $XR_2$ are trans to $P_1$;
$Z_2$ is OH, $C_{1-5}$alkoxy, $-OCH_2CH=CH_2$ or hydrogen,
$Z_1$ is hydrogen;
$R_3$ is hydrogen, $X(CH_2)_nCO_2H$ or $CH=CHCO_2H$;
$R_4$ is hydrogen or $C_{1-2}$alkoxy; and
$R_5$ and $P_2$ are hydrogen.

**4.** A compound according to claim 1 which is:

(1RS, 2SR, 3SR)-1-(4-Methoxyphenyl)-3-(3,4-methylenedioxyphenyl)indane-2-carboxylic acid;
(1RS, 2RS, 3SR)-5-Hydroxy-3-(4-methoxyphenyl)-1-(3,4-methylenedioxyphenyl)indane-2-carboxylic acid;
(1RS, 2RS, 3SR)-5-Methoxy-3-(4-methoxyphenyl)-1-(3,4-methylenedioxyphenyl)indane-2-carboxylic acid;
(1RS, 2RS, 3SR)-1,3-Bis(3,4-methylenedioxyphenyl)-5-hydroxyindane-2-carboxylic acid;
(1RS, 2RS, 3SR)-3-(2-Carboxymethoxy-4-methoxyphenyl)-1-(3,4-methylenedioxyphenyl)-5-(prop-1-yloxy)indane-2-carboxylic acid;
(1RS, 2RS, 3SR)-3-(2-Carboxymethoxy-4-methoxyphenyl)-1-(2-methoxy-4,5-methylenedioxyphenyl)-5-(prop-1-yloxy)indane-2-carboxylic acid;
(1RS, 2RS, 3SR)-3-[2-(1-Carboxyeth-2-yloxy)-4-methoxyphenyl]-1-(3,4-methylenedioxyphenyl)-5-(prop-1-yloxy)indane-2-carboxylic acid;
(1RS, 2RS, 3SR)-3-[2-[(E)-2-Carboxyethen-1-yl]-4-methoxyphenyl]-1-(3,4-methylenedioxyphenyl)-5-(prop-1-yloxy)indane-2-carboxylic acid;
(1RS, 2RS, 3SR)-3-[2-(2-Carboxyeth-1-yl)-4-methoxyphenyl]-1-(3,4-methylenedioxyphenyl)-5-(prop-1-yloxy) indane-2-carboxylic acid;
(1RS, 2RS, 3SR)-3-[2-(3-Carboxyphenyl)-4-methoxyphenyl]-1-(3,4-methylenedioxyphenyl)-5-(prop-1-yloxy) indane-2-carboxylic acid;
or a pharmaceutically acceptable salt of anyone thereof.

**5.** (1RS, 2SR, 3RS)-3-[2-(2-Hydroxyeth-1-yloxy)-4-methoxyphenyl]-1-(3,4-methylenedioxyphenyl)-5-(prop-1-yloxy) indane-2-carboxylic acid; or a pharmaceutically acceptable salt thereof.

**6.** (1RS, 2SR, 3RS)-3-[2-(2-Hydroxyeth-1-yloxy)-4-methoxyphenyl]-1-(3,4-methylenedioxyphenyl)-5-(prop-1-yloxy) indane-2-carboxylic acid, dicyclohexylamine salt.

**7.** A pharmaceutical composition comprising a compound according to any of claims 1 to 6, and a pharmaceutically acceptable carrier.

**8.** A compound according to any of claims 1 to 6 for use as an active therapeutic substance.

**9.** A compound according to any of claims 1 to 6 for use in antagonizing endothelin receptors.

**10.** A compound according to any of claims 1 to 6 for use in treating hypertension, renal failure or cerebrovascular disease.

**11.** Use of a compound according to any of claims 1 to 6 for the manufacture of a medicament for use in the treatment of hypertension, renal failure or cerebrovascular disease.

**12.** A process for the preparation of a compound of Formula (I) as defined in any of claims 1 to 6 or a pharmaceutically acceptable salt thereof, which process comprises reacting a compound of formula (11):

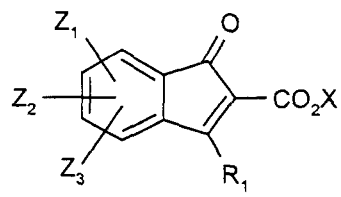

(11)

wherein $Z_1$, $Z_2$, $Z_3$ and $R_1$ are as described in claim 1 or a group convertable thereto, and X is $C_{1-5}$alkyl, with an organomagnesium compound of formula (12):

$$R_2\text{-}(CH_2)_n\text{-}MgBr \qquad (12)$$

wherein $R_2$ is as described in claim 1 or a group convertable thereto, in a suitable solvent to provide a compound of formula (13):

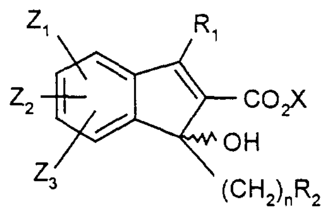

(13)

which is reduced and thereafter, when desired or necessary undergoes,

a) alkylation or acylation to give compounds wherein $P_1$ and $P_2$ are other than $CO_2H$; and/or
b) conversion of $R_1$, $R_2$, $Z_1$, $Z_2$ and $Z_3$ to other groups $R_1$, $R_2$, $Z_1$, $Z_2$ and $Z_3$:

to afford a compound of Formula (I).

**Patentansprüche**

**1.** Verbindung der Formel (I):

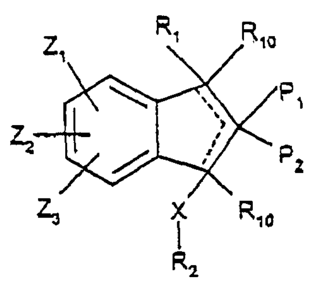

(I)

in welcher $R_1$ für $X(CH_2)_nAr$, eine Dihydrobenzofuranyl-, Benzodioxanyl-, Cyclohexylgruppe oder einen $C_{1-4}$-Alkylrest steht;
$R_2$ eine Einheit der Formel (a) oder (b), einen $C_{1-4}$-Alkylrest, eine Indolylgruppe oder ein Wasserstoffatom bedeutet;
$R_3$ und $R_5$ unabhängig für ein Wasserstoffatom, eine OH-Gruppe, einen $C_{1-5}$-Alkoxyrest, ein Halogenatom, eine -$OC_{1-4}$-Alkylphenylgruppe, $R_{11}CO_2R_7$, einen $C_{1-4}$-Alkylrest, $N(R_6)_2$, $NH(CO)CH_3$, -$X(CH_2)_nR_8$, -X-$R_9$-Y, eine Pyridyl-, Phenylgruppe oder einen $S(O)_qC_{1-5}$-Alkylrest stehen;
$R_4$ ein Wasserstoffatom, eine OH-Gruppe, einen $C_{1-5}$-Alkoxyrest, ein Halogenatom, einen $C_{1-4}$-Alkylrest, $N(R_6)_2$, $NH(CO)CH_3$ oder einen $S(O)_qC_{1-5}$-Alkylrest bedeutet;
$P_1$ und $P_2$ unabhängig für ein Wasserstoffatom, $CO_2H$ oder eine Tetrazolylgruppe stehen;
$R_6$ unabhängig ein Wasserstoffatom oder einen $C_{1-4}$-Alkylrest bedeutet;
$R_7$ unabhängig ein Wasserstoffatom, einen $C_{1-6}$-Alkylrest oder $(CH_2)_nAr$ bedeutet;
$R_8$ für einen $C_{1-8}$-Alkyl-, $C_{2-8}$-Alkenyl- oder $C_{2-8}$-Alkinylrest, die alle unsubstituiert oder durch eine oder mehrere Gruppen OH, $CH_2OH$, $N(R_6)_2$ oder ein Halogenatom substituiert sein können; ein Wasserstoffatom, $CO_2H$, $PO_3H_2$, $P(O)(OH)R_7$ oder eine Tetrazolgruppe steht;
$R_9$ einen $C_{1-10}$-Alkylen-, $C_{2-10}$-Alkenylenrest oder eine Phenylengruppe bedeutet, die alle unsubstituiert oder

durch eine oder mehrere Gruppen OH, $N(R_6)_2$, COOH, ein Halogenatom oder einen $XC_{1-5}$-Alkylrest substituiert sein können;
$R_{10}$ für ein Wasserstoffatom steht;
$R_{11}$ einen $C_{1-8}$-Alkyl-, $C_{2-8}$-Alkenylen- oder $C_{2-8}$-Alkinylenrest bedeutet, die alle unsubstituiert oder durch eine oder mehrere Gruppen OH, $CH_2OH$, $N(R_6)_2$ oder ein Halogenatom substituiert sein können;
X für $(CH_2)_n$ oder ein Sauerstoffatom steht;
Y für eine Gruppe $CH_3$ oder $-X(CH_2)_nAr$ steht;
Ar folgendes bedeutet:

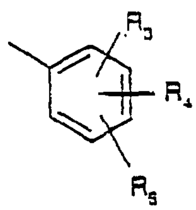
(a),

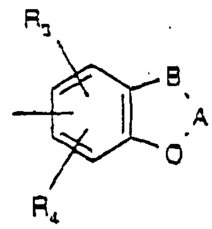
(b),

oder eine Pyridylgruppe, gegebenenfalls durch eine oder mehrere Gruppen $R^3$ oder $R^4$ substituiert;
A für C=O oder $[C(R_6)_2]_m$ steht;
B für $-CH_2-$ oder -O- steht;
$Z_1$, $Z_2$ und $Z_3$ unabhängig für $-X-R_9-Y$, eine Benzylgruppe, ein Wasserstoffatom, eine Gruppe OH, einen $C_{1-5}$-Alkoxyrest, $-N(R_6)_2$, einen $S(O)_qC_{1-8}$-Alkylrest, $NHCOR_6$, $X(CH_2)_nR_8$ oder ein Halogenatom steht, oder $Z_1$ und $Z_2$ zusammen an benachbarten Kohlenstoffatomen -O-A-O- sein können;
q für 0, 1 oder 2 steht;
n eine ganze Zahl von 0 bis 6 bedeutet;
m für 1, 2 oder 3 steht; und die gepunktete Linie das fakultative Vorhandensein einer Doppelbindung angibt; oder ein pharmazeutisch verträgliches Salz davon;

mit der Maßgabe, daß a) wenn die fakultative Doppelbindung vorhanden ist, nur ein $R_{10}$ und kein $P_1$ vorhanden ist; und b) die Verbindung der Formel I nicht folgendes ist:

(1RS)-1,3-Diphenylinden-2-carbonsäure;
(cis,cis)-(1RS,3SR)-1,3-Diphenylindan-2-carbonsäure;
1,3-Diphenylinden;
1,3-Diphenylindan; oder
1,3-Bis(3,4-dimethoxyphenyl)-5,6-dimethoxyindan.

**2.** Verbindung nachAnspruch 1, wobei:

$R_3$ für ein Wasserstoffatom, $-X(CH_2)_nR_8$ oder $R_{11}CO_2R_7$ steht;
$R_4$ und $R_5$ unabhängig für ein Wasserstoffatom, eine Gruppe OH, einen $C_{1-5}$-Alkoxyrest, $SC_{1-5}$-Alkylrest, F, Br, einen $C_{1-3}$-Alkylrest oder $NH_2$ stehen; und
$Z_1$ und $Z_3$ für ein Wasserstoffatom stehen und $Z_2$ ein Wasserstoffatom, eine Gruppe OH, einen $C_{1-5}$-Alkoxyrest, ein Halogenatom, $X(CH_2)_nR_8$, $NH_2$, eine Benzylgruppe oder $NH(CO)CH_3$ bedeuten, oder $Z_1$ und $Z_2$ zusammen an benachbarten Kohlenstoffatomen -O-A-O- sein können.

**3.** Verbindung nach Anspruch 1 oder Anspruch 2, wobei:

$R_1$ für eine Einheit der Formel (b) steht und $R_2$ für eine Einheit der Formel (a) oder (b) steht;
A für $CH_2$ steht;
B für -O- steht;
keine fakultative Doppelbindung vorhanden ist;
$R_1$ und $XR_2$ in trans-Stellung zu $P_1$ stehen;
$Z_2$ für eine Gruppe OH, einen $C_{1-5}$-Alkoxyrest, $-OCH_2CH=CH_2$ oder ein Wasserstoffatom steht;
$Z_1$ für ein Wasserstoffatom steht;

$R_3$ für ein Wasserstoffatom, $X(CH_2)_nCO_2H$ oder $CH=CHCO_2H$ steht;
$R_4$ für ein Wasserstoffatom oder einen $C_{1-2}$-Alkoxyrest steht; und
$R_5$ und $P_2$ ein Wasserstoffatom bedeuten.

**4.** Verbindung nach Anspruch 1, bei welcher es sich um folgende handelt:

(1RS,2SR,3SR)-1-(4-Methoxyphenyl)-3-(3,4-methylendioxyphenyl)indan-2-carbonsäure;
(1RS,2RS,3SR)-5-Hydroxy-3-(4-methoxyphenyl)-1-(3,4-methylendioxyphenyl)indan-2-carbonsäure;
(1RS,2RS,3SR)-5-Methoxy-3-(4-methoxyphenyl)-1-(3,4-methylendioxyphenyl)indan-2-carbonsäure;
(1RS,2RS,3SR)-1,3-Bis(3,4-methylendioxyphenyl)-5-hydroxyindan-2-carbonsäure;
(1RS,2RS,3SR)-3-(2-Carboxymethoxy-4-methoxyphenyl)-1-(3,4-methylendioxyphenyl)-5-(prop-1-yloxy)indan-2-carbonsäure;
(1RS,2RS,3SR)-3-(2-Carboxymethoxy-4-methoxyphenyl)-1-(2-methoxy-4,5-methylendioxyphenyl)-5-(prop-1-yloxy)indan-2-carbonsäure;
(1RS,2RS,3SR)-3-[2-(1-Carboxyeth-2-yloxy)-4-methoxyphenyl]-1-(3,4-methylendioxyphenyl)-5-(prop-1-yloxy)indan-2-carbonsäure;
(1RS,2RS,3SR)-3-[2-[(E)-2-Carboxyethen-1-yl]-4-methoxyphenyl]-1-(3,4-methylendioxyphenyl)-5-(prop-1-yloxy)indan-2-carbonsäure;
(1RS,2RS,3SR)-3-[2-(2-Carboxyeth-1-yl)-4-methoxyphenyl]-1-(3,4-methylendioxyphenyl)-5-(prop-1-yloxy) indan-2-carbonsäure;
(1RS,2RS,3SR)-3-[2-(3-Carboxyphenyl)-4-methoxyphenyl]-1-(3,4-methylendioxyphenyl)-5-(prop-1-yloxy)indan-2-carbonsäure;

oder ein pharmazeutisch verträgliches Salz ingendeiner davon.

**5.** (1RS,2SR,3RS)-3-[2-(2-Hydroxyeth-1-yloxy)-4-methoxyphenyl]-1-(3,4-methylendioxyphenyl)-5-(prop-1-yloxy)indan-2-carbonsäure; oder ein pharmazeutisch verträgliches Salz davon.

**6.** Dicyclohexylaminsalz von (1RS,2SR,3RS)-3-[2-(2-Hydroxye-1-yloxy)-4-methoxyphenyl]-1-(3,4-methylendioxyphenyl)-5-(prop-1-yloxy)indan-2-carbonsäure.

**7.** Arzneimittel, umfassend eine Verbindung nach einem der Ansprüche 1 bis 6 und einen pharmazeutisch verträglichen Träger.

**8.** Verbindung nach einem der Ansprüche 1 bis 6 zur Verwendung als therapeutischer Wirkstoff.

**9.** Verbindung nach einem der Ansprüche 1 bis 6 zur Verwendung zur Antagonisierung von Endothelin-Rezeptoren.

**10.** Verbindung nach einem der Ansprüche 1 bis 6 zur Verwendung bei der Behandlung von Hypertonie, Nierenversagen oder zerebrovaskulärer Verschlußkrankheit.

**11.** Verwendung einer Verbindung nach einem der Ansprüche 1 bis 6 zur Herstellung eines Medikaments zur Verwendung bei der Behandlung von Hypertonie, Nierenversagen oder zerebrovaskulärer Verschlußkrankheit.

**12.** Verfahren zur Herstellung einer Verbindung der Formel (I), wie in einem der Ansprüche 1 bis 6 definiert, oder eines pharmazeutisch verträglichen Salzes davon, wobei das Verfahren das Umsetzen einer Verbindung der Formel (11):

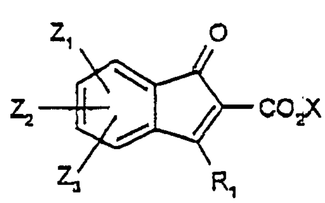

(11)

in welcher $Z_1$, $Z_2$, $Z_3$ und $R_1$ wie in Anspruch 1 beschrieben oder eine in diese umwandelbare Gruppe sind und X

einen $C_{1-5}$-Alkylrest bedeutet, mit einer Organomagnesium-Verbindung der Formel (12):

$$R_2\text{-}(CH_2)_n\text{-}MgBr \qquad (12)$$

in welcher $R_2$ wie in Anspruch 1 beschrieben oder eine in diese umwandelbare Gruppe ist, in einem geeigneten Lösungsmittel umfaßt, um eine Verbindung der Formel (13) bereitzustellen:

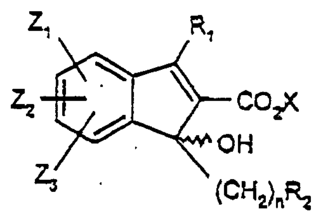

(13)

welche reduziert wird und danach, falls gewünscht oder erforderlich,

a) einer Alkylierung oder Acylierung unter Bereitstellung von Verbindungen, in denen $P_1$ und $P_2$ von $CO_2H$ verschieden sind; und/oder
b) einer Umwandlung von $R_1$, $R_2$, $Z_1$, $Z_2$ und $Z_3$ in andere Gruppen $R_1$, $R_2$, $Z_1$, $Z_2$ und $Z_3$ unter Bereitstellung einer Verbindung der Formel (I)

unterzogen wird.

## Revendications

**1.** Composé de formule (I) :

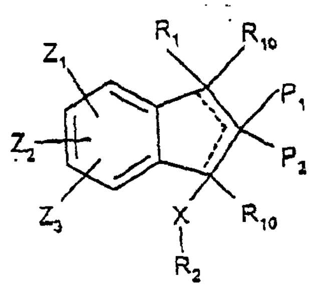

(I)

dans laquelle $R_1$ représente un groupe $X(CH_2)_nAr$, dihydrobenzofurannyle, benzodioxannyle, cyclohexyle ou alkyle en $C_1$ à $C_4$ ;
$R_2$ représente un groupement de formule (a) ou (b), alkyle en $C_1$ à $C_4$, indolyle ou l'hydrogène ;
$R_3$ et $R_5$ représentent indépendamment l'hydrogène, un groupe OH, alkoxy en $C_1$ à $C_5$, halogéno, -O(alkyle en $C_1$ à $C_4$)phényle, $R_{11}CO_2R_7$, alkyle en $C_1$ à $C_4$, $N(R_6)_2$, $NH(CO)CH_3$, $-X(CH_2)_nR_8$, $-X-R_9-Y$, pyridyle, phényle ou $S(O)_q$(alkyle) en $C_1$ à $C_5$) ;
$R_4$ représente l'hydrogène, un groupe OH, alkoxy en $C_1$ à $C_5$, halogéno, alkyle en $C_1$ à $C_4$, $N(R_6)_2$, NH(CO) $CH_3$ ou $S(O)_q$(alkyle en $C_1$ à $C_5$) ;
$P_1$ et $P_2$ représentent indépendamment l'hydrogène, un groupe $CO_2H$ ou tétrazolyle ;
$R_6$ représente indépendamment l'hydrogène ou un groupe alkyle en $C_1$ à $C_4$ ;
$R_7$ représente indépendamment l'hydrogène, un groupe alkylE en $C_1$ à $C_6$ ou $(CH_2)_nAr$ ;

$R_8$ représente un groupe alkyle en $C_1$ à $C_8$, alcényle en $C_2$ à $C_8$ ou alcynyle en $C_2$ à $C_8$, tous ces groupes pouvant être non substitués ou substitués avec un ou plusieurs substituants OH, $CH_2OH$, $N(R_6)_2$ ou halogéno ; l'hydrogène, un groupe $CO_2H$, $PO_3H_2$, $P(O)(OH)R_7$ ou tétrazole ;
$R_9$ représente un groupe alkylène en $C_1$ à $C_{10}$, alcénylène en $C_2$ à $C_{10}$ ou phénylène, tous ces groupes pouvant être non substitués ou substitués avec un ou plusieurs substituants OH, $N(R_6)_2$, COOH, halogéno ou X(alkyle en $C_1$ à $C_5$) ;
$R_{10}$ représente l'hydrogène ;
$R_{11}$ représente un groupe alkylène en $C_1$ à $C_8$, alcénylène en $C_2$ à $C_8$ ou alcynylène en $C_2$ à $C_8$, tous ces groupes pouvant être non substitués ou substitués avec un ou plusieurs substituants OH, $CH_2OH$, $N(R_6)_2$ ou halogéno ;
X représente un groupe $(CH_2)_n$ ou l'oxygène ;
Y représente un groupe $CH_3$ ou $-X(CH_2)_nAr$ ;
Ar représente : un groupe

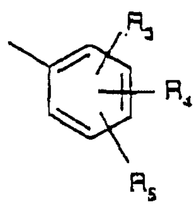
(a),
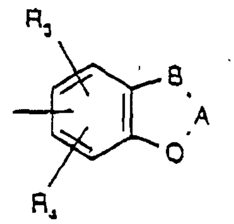
(b),

ou pyridyle facultativement substitué avec un ou plusieurs groupes $R^3$ ou $R^4$ ;
A représente un groupe C=O ou $[C(R_6)_2]_m$ ;
B représente un groupe $-CH_2-$ ou -O- ;
$Z_1$, $Z_2$ et $Z_3$ représentent indépendamment un groupe
$-X-R_9-Y$, benzyle, l'hydrogène, un groupe OH, alkoxy en $C_1$ à $C_5$, $-N(R_6)_2$, $S(O)_q$(alkyle en $C_1$ à $C_8$), $NHCOR_6$, $X(CH_2)_nR_8$ ou halogéno, ou bien $Z_1$ et $Z_2$, conjointement, peuvent représenter un groupe -O-A-O- sur des atomes de carbone contigus ;
q est égal à zéro, un ou deux ;
n représente un nombre entier de 0 à six ;
m est égal à 1, 2 ou 3 ; et la ligne discontinue indique la présence facultative d'une double liaison ou un de ses sels pharmaceutiquement acceptables ;
sous réserve que a) lorsque la double liaison facultative est présente, il existe un seul groupe $R_{10}$ et il n'existe aucun groupe $P_1$ ; et b) le composé de formule I ne soit pas :

l'acide (1RS)-1,3-diphénylindène-2-carboxylique ;
l'acide (cis, cis)-(1RS,3SR)-1,3-diphénylindane-2-carboxylique ;
le 1,3-diphénylindène ;
le 1,3-diphénylindane ; ou
le 1,3-bis(3,4-diméthoxyphényl)-5,6-diméthoxyindane.

**2.** Composé suivant la revendication 1, dans lequel :

$R_3$ représente l'hydrogène, un groupe $-X(CH_2)_nR_8$ ou $R_{11}CO_2R_7$ ;
$R_4$ et $R_5$ représentent indépendamment l'hydrogène, un groupe OH, alkoxy en $C_1$ à $C_5$, S (alkyle en $C_1$ à $C_5$), F, Br, alkyle en $C_1$ à $C_3$ ou $NH_2$ ; et
$Z_1$ et $Z_3$ représentent l'hydrogène et $Z_2$ représente l'hydrogène, un groupe OH, alkoxy en $C_1$ à $C_5$, halogéno, $X(CH_2)_nR_8$, $NH_2$, benzyle ou $NH(CO)CH_3$ ou bien $Z_1$ et $Z_2$, conjointement, peuvent représenter un groupe -O-A-O- sur des atomes de carbone contigus.

**3.** Composé suivant la revendication 1 ou la revendication 2, dans lequel :

$R_1$ représente un groupement de formule (b) et $R_2$ représente un groupement de formules (a) ou (b) ;
A représente un groupe $CH_2$ ;
B représente un groupe -O- ;
il n'existe aucune double liaison facultative ;
$R_1$ et $XR_2$ sont en positions trans par rapport à $P_1$ ;
$Z_2$ représente un groupe OH, alkoxy en $C_1$ à $C_5$, $-OCH_2CH=CH_2$ ou l'hydrogène,
$Z_1$ représente l'hydrogène ;
$R_3$ représente l'hydrogène, un groupe $X(CH_2)_nCO_2H$ ou $CH=CHCO_2H$ ;
$R_4$ représente l'hydrogène ou un groupe alkoxy en $C_1$ ou $C_2$ ; et
$R_5$ et $P_2$ représentent l'hydrogène.

**4.** Composé suivant la revendication 1, qui est :

l'acide (1RS, 2SR, 3SR)-1-(4-méthoxyphényl)-3-(3,4-méthylènedioxyphényl)indane-2-carboxylique ;
l'acide (1RS, 2RS, 3SR)-5-hydroxy-3-(4-méthoxyphényl)-1-(3,4-méthylènedioxyphényl)-indane-2-carboxylique ;
l'acide (1RS, 2RS, 3SR)-5-méthoxy-3-(4-méthoxyphényl)-1-(3,4-méthylènedioxyphényl)-indane-2-carboxylique ;
l'acide (1RS, 2RS, 3SR)-1,3-bis(3,4-méthylènedioxyphényl)-5-hydroxyindane-2-carboxylique ;
l'acide (1RS, 2RS, 3SR)-3-(2-carboxyméthoxy-4-méthoxyphényl)-1-(3,4-méthylènedioxyphényl)-5-(prop-1-yloxy)indane-2-carboxylique ,
l'acide (1RS, 2RS, 3SR)-3-(2-carboxyméthoxy-4-méthoxyphényl)-1-(2-méthoxy-4,5-méthylènedioxyphényl)-5-(prop-1-yloxy)indane-2-carboxylique ;
l'acide (1RS, 2RS, 3SR)-3-[2-(1-carboxyéth-2-yloxy)-4-méthoxyphényl]-1-(3,4-méthylènedioxyphényl)-5-(prop-1-yloxy)indane-2-carboxylique ;
l'acide (1RS, 2RS, 3SR)-3-[2-[(E)-2-carboxyéthèn-1-yl]-4-méthoxyphényl]-1-(3,4-méthylènedioxyphényl)-5-(prop-1-yloxy)indane-2-carboxylique ;
l'acide (1RS, 2RS, 3SR)-3-[2-(2-carboxyéth-1-yl)-4-méthoxyphényl]-1-(3,4-méthylènedioxyphényl)-5-(prop-1-yloxy)indane-2-carboxylique ;
l'acide (1RS, 2RS, 3SR)-3-[2-(3-carboxyphényl)-4-méthoxyphényl]-1-(3,4-méthylènedioxyphényl)-5-(prop-1-yloxy)indane-2-carboxylique ;

ou un sel pharmaceutiquement acceptable de l'un quelconque de ces composés.

**5.** Acide (1RS, 2SR, 3RS) -3-[2-(2-hydroxyéth-1-yloxy)-4-méthoxyphényl]-1-(3,4-méthylènedioxyphényl)-5-(prop-1-yloxy)indane-2-carboxylique ; ou un de ses sels pharmaceutiquement acceptables.

**6.** Sel de dicyclohexylamine d'acide (1RS, 2SR, 3RS)-3-[2- (2-hydroxyéth-1-yloxy) -4-méthoxyphényl] -1- (3,4-méthylènedioxyphényl) -5- (prop-1-yloxy) indane-2-carboxylique.

**7.** Composition pharmaceutique comprenant un composé suivant l'une quelconque des revendications 1 à 6, et un support pharmaceutiquement acceptable.

**8.** Composé suivant l'une quelconque des revendications 1 à 6, destiné à être utilisé comme substance thérapeutique active,

**9.** Composé suivant l'une quelconque des revendications 1 à 6, destiné à être utilisé pour antagoniser les récepteurs d'endothéline.

**10.** Composé suivant l'une quelconque des revendications 1 à 6, destiné à être utilisé dans le traitement de l'hypertension, d'insuffisance rénale ou d'une maladie vasculaire cérébrale.

**11.** Utilisation d'un composé suivant l'une quelconque des revendications 1 à 6 pour la production d'un médicament destiné à être utilisé dans le traitement de l'hypertension, de l'insuffisance rénale ou d'une maladie vasculaire cérébrale.

**12.** Procédé pour la préparation d'un composé de formule (I) répondant à la définition suivant l'une quelconque des revendications 1 à 6 ou un de ses sels pharmaceutiquement acceptables, procédé qui comprend la réaction d'un

composé de formule (11) :

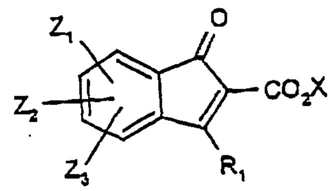

(11)

dans laquelle $Z_1$, $Z_2$, $Z_3$ et $R_1$ représentent des groupes répondant à la définition figurant dans la revendication 1 ou des groupes pouvant être convertis en de tels groupes, et X représente un groupe alkyle en $C_1$ à $C_5$, avec un composé organique de magnésium de formule (12) ;

$$R_2\text{-}(CH_2)_n\text{-}MgBr \qquad (12)$$

dans laquelle $R_2$ représente un groupe répondant à la définition figurant dans la revendication 1 ou un groupe pouvant être converti en un tel groupe, dans un solvant convenable pour produire un composé de formule (13) :

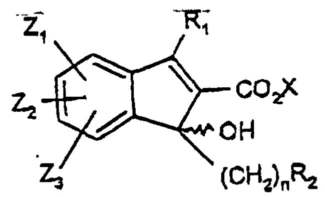

(13)

qui est réduit et subit ensuite, lorsque cela est- désiré ou nécessaire,

a) une alkylation ou acylation pour obtenir des composés dans lesquels $P_1$ et $P_2$ sont autres que des groupes $CO_2H$ ; et/ou
b) une conversion des groupes $R_1$, $R_2$, $Z_1$, $Z_2$ et $Z_3$ en d'autres groupes $R_1$, $R_2$, $Z_1$, $Z_2$ et $Z_3$ ;

ce qui donne un composé de formule (I).